(19) Europäisches Patentamt
European Patent Office
Office européen des brevets

(11) Publication number: 0 145 486 A2

# (12) EUROPEAN PATENT APPLICATION

(21) Application number: 84308620.8

(22) Date of filing: 12.12.84

(51) Int. Cl.⁴: C 12 N 15/00, C 12 P 21/02, C 12 N 1/20, A 61 K 39/106, A 61 K 39/108

(30) Priority: 12.12.83 GB 8333131

(43) Date of publication of application: 19.06.85 Bulletin 85/25

(84) Designated Contracting States: AT BE CH DE FR GB IT LI LU NL SE

(71) Applicant: GLAXO GROUP LIMITED, Clarges House 6-12 Clarges Street, London W1Y 8DH (GB)

(72) Inventor: Hayes, Michael Victor, 54 Wimborne Drive, Pinner, Middlesex (GB)
Inventor: Harford, Stephen, 50 Bourne View Greenford, Middlesex (GB)
Inventor: Ross, Gordon William, 35 Brookdene Drive Northwood, Middlesex (GB)

(74) Representative: Holmes, Michael John et al, Frank B. Dehn & Co. European Patent Attorneys Imperial House 15-19 Kingsway, London, WC2B 6UZ, (GB)

## (54) Microbiological process.

(57) A toxoid comprising an inactive form of a toxin or toxic component of a toxin, which inactivate form is substantially homologous with said toxin or toxic component, is produced by expression of a modified gene and is substantially non-toxic while being, alone or in combination with one or more non-toxic components, capable of stimulating the production of antibodies against the said toxin or toxic component is described. The toxoid may be used in vaccine preparations. A toxoid has been developed which is of use in the treatment of pig scours. A method of producing the toxoid is described using recombinant DNA technology.

EP 0 145 486 A2

FB 146-917D

# Microbiological Process

This invention relates to novel vaccines containing inactivated toxins.

Many diseases in humans and animals are the result of toxin production by pathogenic bacteria, the toxins disrupting the normal cellular activity. The body does, however, have a defence system in the form of the immune response, whereby antibody molecules are produced in response to the introduction of antigenic proteins, e.g. bacterial toxins or proteins present on the exterior of the bacteria themselves. A specific antibody is produced in response to the antigenic determinants of the protein, and may persist in the blood for some time. Therefore, if the antigenic protein should later appear in the blood or lymph, the antibody forming cells that were primed by a previous exposure to the antigen rapidly produce specific antibodies to eliminate it. This concept is the basis of many vaccination techniques, whereby a small quantity of a relatively harmless form of the antigen itself is administered to condition the cells to produce the required antibody.

Pathogenic bacteria themselves are not usually suitable for use in a vaccine because they have the potential to cause disease. The pathogen itself therefore is normally killed, e.g. by heating, so that full scale infection cannot occur. In view of the necessity to kill all the bacteria, a high degree of quality control is necessitated in manufacturing the vaccine. This system also requires growth of large amounts of pathogenic bacteria, which are potential infectants of those involved in vaccine production, and care must also be taken to ensure that live pathogens do not escape to the surrounding environment. Furthermore, chemically or physically inactivated bacterial vaccines may not afford the same degree of protection as live pathogens or their toxins themselves.

If toxins are used, they too must be inactivated, generally by use of formaldehyde, to yield 'toxoids'. Again, the problem of quality control arises, since the existence of unmodified toxins in the vaccine may lead to severe illness and possibly death.

The present invention provides a new approach to vaccination using the techniques of recombinant DNA technology. We have found that it is possible to modify the gene coding for a toxin or a toxic component of a toxin in such a way that when incorporatec into a suitable vector, if necessary together with other genetic material, and used to transform a organism, it produces a toxoid, i.e. a modified toxin which is capable of stimulating production of antibodies active against the natural toxin or toxic component of the toxin, while no longer being toxic. The genetically modified toxin so produced may then be incorporated into a vaccine alone or with other antigens or in the form of transformed non-pathogenic host organisms containing the modified toxin.

According to the present invention therefore there is provided a toxoid comprising an inactivated form of a toxin or toxic component of a toxin, which inactivated form is substantially homologous with said toxin or toxic component, is produced by expression of a modified gene and is substantially non-toxic while being, alone or in combination with one or more non-toxic components, capable of stimulating the production of antibodies against the said toxin or toxic component of the toxin.

As indicated above, the inactivated toxin or component is substantially homologous with the natural toxin or component, that is it preferably differs only by a small number, for example less than 10, preferably less than 5 e.g. 1 or 2, amino acid residues at or close to a site necessary for toxic activity. It is particularly preferred that this inactive form of the toxin or toxic component conserves the spatial relationship between the amino acid residues which are common to both the active toxin or toxic component and the said inactive form, so that toxic activity is removed while retaining as much as possible of the original immunogenicity.

According to a further aspect of the invention we provide a method of inactivation of a toxin or toxic component of a toxin wherein a gene coding for the toxin or a toxic component of the toxin is modified in such a way that when the modified gene, if necessary with other genes, is incorporated into a vector capable of replication in a host organism, and used to transform said host organism it produces a toxoid according to the invention as defined above.

The invention also extends to a gene modified to code for an inactivated toxin or component of a toxin according to the invention. Such a gene is preferably substantially homologous with the gene coding for the natural toxin or toxic component. The invention also includes vectors such as plasmids containing such modified genes.

There are two mutagenic techniques which may advantageously be employed to modify a gene coding for a toxin causative of a particular disease. The first employs random mutagenesis, for example using a chemical mutagen or irradiation. The randomly modified genes so obtained are then screened for those producing a modified toxin having the desired properties.

The second approach involves site directed mutagenesis by modifying the region of the gene coding for a site in the corresponding protein necessary for toxic activity (i.e. active region). This may be achieved for example by introduction of an oligonucleotide, conveniently a chemically synthesised oligonucleotide, at that position, so that the gene codes for a closely related, substantially homologous, protein which no longer has a toxic effect, but still gives rises to antibody production.

According to one embodiment of this technique where there is an available restriction endonuclease which will cut the gene at a point in the active region, it is possible to insert an oligonucleotide at that point to produce a gene coding for a slightly larger protein having the desired properties.

Where the nucleotide sequence of the gene coding for an active region of the protein has been determined and if it contains two restriction sites the opportunity also exists for removal of the sequence between the two sites and either joining the cut ends so formed or inserting a selected oligonucleotide differing only slightly from the sequence removed. In the latter case, the oligonucleotide may be larger or smaller than the removed sequence and/or differ at one or more positions.

Another method of site directed mutagenesis is primer directed mutagenesis, using a single stranded DNA template. In this method the nucleotide sequence of at least the part of the gene coding for the active region of the toxin has to be determined. A single stranded form of the gene is obtained either by strand separation or by cloning into single stranded phage. An oligonucleotide (usually 14-20 nucleotides in length) can be synthesised that has a sequence that is complementary to the "active" region of the gene. This oligonucleotide can be used to prime the synthesis of the entire complementary strand using the single stranded material as a template. This method of introducing a mutation relies on the structure of the oligonucleotide primer. If the oligonucleotide primer is made in such a way as to be complementary to a given region of the gene but with a small variation, preferably a single mismatch in the base to be altered, then this molecule will act as a primer to direct the synthesis of the second strand. The duplex of DNA so formed will have two strands which are complementary except at the point into which the specific mutation has been directed. More than one mismatch may be present but the oligonucleotide primer must be sufficiently homologous with the natural "active" site to permit synthesis of the complementary strand. It will be appreciated that the replication of this molecule will yield two heterologous double stranded DNA molecules differing in a single base-pairing. The modified DNA may for example be detected and isolated using a labelled form of the oligonucleotide primer as a probe since it will bind more strongly to the modified than to the unmodified DNA.

It will be appreciated that in all such specific mutations, the new oligonucleotide sequence must maintain the reading frame and must not contain undesirable codons such as stop codons.

Many gastro-enteric diseases caused by gram-negative bacteria are characterised by acute diarrhoea, which is commonly the result of toxin production leading to a disruption of fluid balance within the gut. Scours is an example of such a disease in pigs and cattle, causing acute diarrhoea especially in young animals, while cholera is a particularly notable example of a similar disease in humans. The disease is commonly the result of contamination and colonisation of the small intestine by enterotoxigenic strains of Escherichia coli in the case of scours and Vibrio cholerae in the case of cholera.

It has been shown that colonisation of the small intestine by bacteria is initiated by bacterial attachment to the host intestinal wall via an antigenic protein located on the bacterial cell surface, for example the so-called K88 antigen in the case of pig scours and K99 antigen in the case of cattle scours. Many previous approaches to vaccination against diseases causing acute diarrhoea have involved development of antibodies against these surface antigens, but these approaches have not employed inactivation of the bacterial toxins.

The present invention relates to development of a vaccine which stimulates antibody formation against a toxin which is reponsible for the acute diarrhoea produced by the infection. In cholera this is normally termed CT while in pig scours a related heat labile toxin (LT) is responsible for acute diarrhoea and high mortality.

In the case of pig scours the LT toxin is composed of two proteins, LTA and LTB, in a ratio of 1:5, which have different functions. The LTB protein (which is situated externally) binds the toxin to the intestinal wall and then the LTA protein, which is surrounded by the LTB protein, is secreted and penetrates the epithelial membrane of the gut where it stimulates the activity of adenyl cyclase. This enzyme stimulation leads directly to fluid secretion into the gut and to the development of severe diarrohea. Similarly, cholera toxin comprises components CTA, which is responsible for adenyl cyclase stimulation, and CTB, which binds the toxin to the intestinal wall.

Previous investigations into protection against pig scours and similar diseases have, in addition to concentrating on bacterial adhesion factors, focused on vaccines containing only the external non-toxic protein component of the toxin, e.g. the above LTB protein. However, it is believed that a vaccine containing both LTA and LTB is more immunogenic than one containing the LTB component alone since in studies involving protection of dogs against cholera toxin, the CTB component, analogous to LTB, has been shown to be forty times less efficient than whole CT in providing protection.

It has also been proposed (EPA 0087735) to modify the LTA toxin by removing a large part of the molecule, e.g. about one third of its length, in order to remove its toxic activity, while retaining some of its immunological properties. However, the modified toxin so formed is not capable of combination with LTB to form a modified LT toxin and inevitably loses many of the antigenic determinants responsible for the immunounimological properties of the toxin. We have found that it is preferable to adopt a different approach, namely that set out above whereby the toxin is changed as little as possible and remains substantially homologous with the natural LTA toxin, thus retaining virtually all the antigenic determinants of the original molecule.

According to a further aspect of the invention there is provided a toxoid comprising a genetically modified form of the toxic component of a toxin causing acute diarrhoea which is substantially homologous with the toxic component and is substantially non-toxic but, either alone or in combination with a further non-toxic component stimulates the production of antibodies active against the natural toxin or toxic component. The invention further provides a method of altering a gene coding for such a toxic component in such a way that it codes for a toxoid as defined above.

The production of the modified toxic component necessitates isolation of the gene coding for that component (hereinafter called the A-gene) normally associated with a gene coding for the non-toxic component of the toxin (hereinafter called the B-gene). The gene may be located in a plasmid, e.g. in the case of pig scours, P307, which may be isolated from E.coli NCIB 11932. The gene modification process cannot conveniently be carried out on the original plasmid in view of its size and lack of recognition characteristics. The selection of clones containing the modified gene is facilitated if it is isolated and inserted into a more suitable vector.

Thus, in the case of pig scours, the original plasmid P307 may be digested with EcoRl and Hpal and the digested fragments mixed with the specially developed plasmid pAT153 described hereinafter (which has previously been digested to completion with EcoRl and partially digested with HincII) and ligated. The hybrid plasmids so formed may be used to transform a suitable host organism, such as E.coli NCIB 11933. The successful transformants may be selected on the basis of production of LTA protein and the plasmids may be isolated by gel electrophoresis. In this way we have been able to identify the transformant containing plasmid GpELTA-6 coding for LTA protein.

The B-gene may conveniently also be isolated and inserted into a plasmid to enable expression of the LTB-component of the toxin. In the case of pig scours the plasmid P307 may be digested with Hind III and the digested fragments mixed with plasmid pAT153 (which has previously been digested with Hind III followed by dephosphorylation) and ligated. Transformation of E. coli NCIB 11933 and cloning produce one or more transformants producing LTB protein and the responsible plasmid may be analysed by gel electrophoresis. In this way, we have identified plasmid GpELTB-10 coding for LTB protein.

The next step is to modify the A-gene so that the protein product is non-toxic whilst still capable of stimulating production of antibodies. It is also desirable that the protein product should associate with the B component as in the natural toxin upon expression of the modified A gene in conjunction with the B gene. We have applied the strategies mentioned above to produce such genes.

The first technique thus involves modification of the A-gene by treatment of the plasmid containing the inserted A-gene, for example the GpELTA-6 plasmid, conveniently with a mutagen, for example hydroxylamine or a bisulphite (e.g. sodium or potassium bisulphite). The mutagenised plasmid is then used to transform a non-pathogenic E.coli strain such as strain NCIB 11933. Transformants may then be selected on the basis of the ability of their cell free extracts to produce a protein product that will, upon expression in conjunction with the B gene, give a non-toxic immunogenic protein of a similar molecular weight to the natural toxin.

In order to produce a modified toxin containing both A- and B-components, the separate plasmids containing the A- and B-genes may be cut e.g. by a restriction enyzme such as EcoRl, and joined to produce a hybrid plasmid. Thus, we have joined plasmids GpELTA-6ml, -6m2 and -6m3 respectively to plasmid GpELTB-10 following digestion by Eco Rl, and used the three resulting hybrid plasmid to transform E. coli NCIB 11933. The three new plasmids were characterised by genetic mapping and named GpENT-2ml, GpENT-2m2 and GpENT-2m3. In addition, the original GpELTA-6 plasmid was joined to the same fragment from GpELTB-10 to produce the plasmid

GpENT-2. After cloning the three transformed strains of NCIB 11933, the modified toxins were then isolated and found to consist of LTA and LTB in a ratio of 1:5 in all cases, identical to the ratio found in the natural LT toxin.

Using the same technique we have prepared further plasmids containing modified LTA genes, both with and without the gene for the LTB toxin component. Following the above nomenclature these have been termed GpENT-2m4 to GpENT-2m11 and GpELTA-6m4 to GpELTA-6m11 respectively.

The gene sequence of the natural LTA gene hereinbefore described is shown is Figure 1 of the accompanying drawings. The modifications present in GpENT-2m1 to GpENT-2m11, described above have been ascertained and are shown in Table 1 together with corresponding amino acid sequences.

TABLE 1

| MUTANT GpENT-2m | AMINO-ACID POSITION | NUCLEOTIDE CHANGE FROM | NUCLEOTIDE CHANGE TO | AMINO ACID CHANGE FROM | AMINO ACID CHANGE TO |
|---|---|---|---|---|---|
| 1 | 79 | TCC | TTT | Ser | Phe |
| | 97 | GGA | AAA | Gly | Lys |
| 2 | 100 | ACT | ATT | Thr | Ile |
| | 101 | TAC | TAT | Tyr | Tyr |
| | 151 | GGT | GAT | Gly | Asp |
| 3 | 101 | TAC | TAT | Tyr | Tyr |
| | 110 | CCA | CTA | Pro | Leu |
| | 171 | CTG | CTA | Leu | Ley |
| 4 | 93 | TCT | TTT | Ser | Phe |
| 5 | 138 | CCA | CTA | Pro | Leu |
| 6 | 136 | GGA | GAA | Gly | Glu |
| 7 | 39 | GGT | GAT | Gly | Asp |
| | 165 | GAC | AAC | Asp | Asn |
| 8 | 112 | ATG | ATA | Met | Ile |
| | 115 | GTT | ATT | Val | Ile |
| | 118 | GTA | ATA | Val | Ile |
| 9 | 135 | GGT | AGT | Gly | Ser |
| | 158 | CAT | TAT | His | Tyr |
| 10 | 32 | GAT | AAT | Asp | Asn |
| | 96 | TCA | TTA | Ser | Leu |
| 11 | 44 | GGG | AGA | Gly | Arg |

ALL MUTANTS LTA* i.e. SHOW NO ACTIVITY IN THE LTA ASSAY (AS IN EXAMPLE 1B (VI)

The activity of the toxoids and the natural toxin was tested by the rabbit ileal loop test using cell free extracts of bacterial strains containing the relevant plasmids. We found that the protein products of GpENT-2ml-3 to GpENT-2ml1 and GpELTA-6ml to GpELTA-6ml1 had no measureable toxic activity.

As mentioned above, the second strategy for inactivation of the gene, in this case the A-gene, involves specific mutagenesis using a defined oligonucleotide to modify the site coding for the section of the A-component of the toxin responsible for toxic activity. This modification of the DNA sequence results in a protein altered in a region necessary for toxic activity. It is, of course, preferable to find a restriction site in or sufficiently close to the sequence of the gene coding for that region. A suitable restriction endonuclease can be found by experiment; if necessary plasmids may have to be specially chosen which do not possess sites recognised by this particular restriction enzyme.

The oligonucleotide insert desirably contains a recognition site facilitating selection of clones containing the modified gene. This may conveniently be a restriction site not occuring elsewhere in the plasmid; modified and unmodified plasmids can then be distinguished by treatment with the restriction enzyme and subjecting the products to gel electrophoresi to distinguish linear from circular DNA. The oligonucleotide must be chosen so as to maintain the reading frame and must not contain a stop codon. It is convenient to produce the oligonucleotide containing such a site by chemical synthesis since relatively short sequences are effective. However, if a naturally occuring sequence having the required properties exists this could be used.

The synthetic oligonucleotide may be prepared, for example, using the established phosphotriester procedure of M.Gait _et al_ (Chemical and Enzymatic Synthesis of Gene fragments, Ed. H.G. Gassen and A. Long; Verlag Chemie Weinheim 1982). It is preferred to use a short oligonucleotide, for example one coding for 3 to 5 amino acids. When using the pAT153 plasmid, one possible unique recognition site is one recognising the restriction endonuclease Sst II.

It will be appreciated that the oligonucleotide(s) should be complimentary in such a way that they form dimers having adhesive termini corresponding to the cut ends of the gene into which they are to be inserted. Thus for example, we have synthesised the oligonucleotide d(CTAGTCCGCGGA) which forms the dimer

5' CTAGTCCGCGGA
AGGCGCCTGATC 5'

We were able to cut the plasmid GpELTA-6 using Xba 1 and insert the above synthetic oligonucleotide d(CTAGTCCGCGGA) phosphorylated at the 5' terminus. The modified plasmid was then used to transform E.coli NCIB 11933 and cloned. The analysis of the plasmid/oligonucleotide hybrids showed that there were a number of mutants which had lost the sequence of DNA recognised by Xba 1, and gained a site recognised by Sst II, implying successful insertion of the oligonucleotide at this site. These mutants were analysed by the Zubay in vitro transcription/translation system. This showed that seven of the mutants (GpELTA 6s 1-7) produced proteins coded by the modified LTA genes.

The next step involved construction of a plasmid coding for the complete toxoid. This was carried out as described earlier, by inserting the LTB gene derived from GpELTB-10 into one of the plasmids containing the insertion -inactivated LTA, for example GpELTA 6s5. A new plasmid was formed, GpENT-2s5.

In order to test the protein product of the newly generated GpENT-2s5, cell free extracts of bacterial strains transformed by GpENT-2 (containing the natural LTA and LTB genes) and GpENT-2s5 (containing the natural LTB gene and the modified LTA gene) were tested for activity in the rabbit ileal loop. It was found that there was no response to GpENT-2s5, showing the protein product was inactive. It can therefore be concluded that oligonucleotide insertion at the Xba 1 site of the LTA gene results in the production of a new, slightly larger, protein, which is no longer toxic.

We have also produced an LT-toxoid by primer-directed oligonucleotide mutagenesis based, as described above, on the construction of an oligonucleotide complimentary to a given region of the LTA gene but with a slight modification, this oligonucleotide then acting as a primer to direct synthesis of a complimentary strand which is altered so as to code for an inactive LTA peptide.

The synthetic oligonucleotide may be constructed as above. We have, for example, synthesised the oligonucleotide

TGTTTTCACTTCTCTTAG

which differs by only one base from the oligonucleotide TGTTTCCACTTCTCTTAG

present in the native gene.

This synthetic oligonucleotide may then be phosphorylated and used to prime single stranded DNA which carries the complimentary strand of the LTA gene. The DNA vector used is one which is capable of existing in single stranded form, as a phage, while being able to replicate as a double stranded form in a host bacterium. One example of such a vector is M13mp10. We have thus initially prepared an M13mp10/LTA hybrid vector by digesting M13mp10 with Eco R1 and Xba 1, using these same endoncleases to digest GpELTA-6, and then ligating the two fragments together. The hybrid may be identified following transformation into an E.coli, e.g. JM103. An E.coli containing this hybrid may then be used to produce phage particles which contain the hybrid in single stranded form. The primer oligonucleotide may then be annealed to the single stranded template and used to generate a second strand using the action of DNA polymerase. This double stranded molecule is then ligated and cloned into a suitable host organism, e.g. E.coli JM103. On replication of this double stranded DNA, two heterologous double stranded DNA molecules are produced, one of which codes for the natural toxin while the other codes for the toxoid. Cells containing the modified gene encoding for the toxoid may then be identified.

The modified genes produced by the above techniques may be used to produce the desired toxoid by incorporation into suitable plasmids as discussed above. In this way we have prepared the plasmid GpENT3SDM1 containing the gene modified as above. The plasmid may then be used to transform appropriate host cells, such as E.coli NCIB 11933, which may then be subjected to conventional fermentation techniques and the toxoid isolated.

Thus, for example, the transformed host cells may be cultured by fermentation on or in a nutrient medium therefor, which will normally contain a source of nitrogen, such as a protein hydrolysate, e.g. peptone, or one or more ammonium salts; a source of carbon and energy such as Lab lemco or glycerol, and trace elements. In the case of E.coli, submerged aerobic fermentation is preferred, advantageously at about 37°C.

The toxoid may be isolated from the fermentation in any convenient way. In general, the transformed host cells will not be capable of expressing the toxoid extracellularly. Consequently, the cells will normally be collected, e.g. by centrifugation; and disrupted, e.g. by sonication or lysis, and the extract, after removal of cell debris, subjected to fractionation, for example fractional precipitation or chromatography. Thus, for example, LT toxoid may be purified by application of such a cell-free extract to an agarose column and elution with galactose in TEAN (50mM Tris-HCl 50mM EDTA, 200mM NaCl, 3mM sodium azide, pH 7.5).

If required, the toxoid may be lyophilised for storage, prior to incorporation into a vaccine formulation.

In general, it is preferred to modify the gene so as to produce modifications between nucleotide residues Nos. 229 (corresponding to the start of the DNA triplet coding for Tyr) and 459 (corresponding to the end of the DNA coding for Val). The LTA protein is thus preferably modified between amino acids 77 (Tyr) and 153 (Val).

It will be appreciated that the degeneracy of the genetic code allows more than one coding triplet to code for a particular amino acid, and the invention extends to all genes equivalent to those shown in Figure 1 according to the well-known rules of degeneracy. (The Genetic Code, Cold Spring Harbor Symp. Quant. Biol., vol 31, 1966).

Therefore, according to a further aspect of the invention we provide modified genes coding for the LTA toxoid according to the invention and, more particularly, genes modified as shown in Figure 1 and their equivalents. The invention extends to a gene coding for the LTA subunit of LT toxin causative of pig scours wherein the nucleotide triplet coding for serine at position No. 79 is replaced by a triplet coding for phenylalanine and up to two further triplets are replaced by triplets coding for different amino acids.

The invention also extends to vectors containing such modified genes. In the case of LTA genes, these are preferably present in a plasmid in conjunction with a gene coding for the LTB protein and in general where a gene coding for a toxic component of a toxin causing acute diarrhoea is concerned, it is preferably present in a plasmid together with a gene coding for the remainder of the toxin (the B gene) so that a complete toxoid is expressed. Particularly preferred plasmids are those derived from pAT153, especially GpENT-2m1, GpENT-2m2 and GpENT-2m3.

The invention also extends to host cells transformed with such plasmids, especially E.coli cells such as NCIB 11933. Where the cells are to be incorporated into a vaccine it is important that they should not only be non-pathogenic but that they should not generate pyrogens. E.coli NCIB 11933 cells are suitable for this purpose.

A further aspect of this invention relates to vaccine preparations containing a toxoid according to the invention. Such a toxoid may be present in purified or partially purified form or may be contained in a non-pathogenic host cell which has been used to produce it. We particularly provide a vaccine against scours disease containing the complete toxoid hereinbefore described, wherein the modified LTA component is combined with the LTB component in the 1:5 ratio found in the natural toxin, which may be present, as indicated above, in non-pathogenic host cells or in purified or partially purified form.

The vaccine used against pig scouring may desirably incorporate non-pathogenic host cells containing the toxoid or purified or partially purified toxoid, together with whole bacteria containing K88 antigens or purified or partially purified K88 antigens. Plasmids coding for the K88 antigens used, namely K88 types ab, ac and ad, and transformed cells containing them have been described, for example, in EP. 0060129. Corresponding adhesion factors, such as K99 in the case of cattle scours, may suitably be substituted for K88 antigens to produce a vaccine effective against related diseases.

Where it is desired to use isolated K88 antigens these can be recovered after fermentation of host cells containing genes coding for such antigens by the methods described by Stirm et al (J. Bacteriol. (1967) 93, 731 - 739). Heat-treatment of the cells, isoelectric precipitation and membrane filtration are particularly useful techniques in their recovery. Where a proportion or all of the antigens are released into the culture fluid, either as a consequence of the fermentation conditions, or by heating the fermentation broth at the end of the fermentation, the cell-free antigen can be separated from the cells by conventional means, such as centrifugation, filtration or ultrafiltration. The antigens can subsequently be recovered by the methods described by Stirm et al.

The vaccine may be formulated in a suitable adjuvant. Such adjuvants, whose purpose is to potentiate the immune response, include, for example, gel forming aluminium compounds (e.g. aluminium, hydroxide or aluminium phosphate) or water-in-oil emulsions, such as Freund's complete adjuvant or, more preferably, emulsions comprising purified paraffin oil, an oil soluble surfactant, water and a water-soluble surfactant, the ratio of oil to water being 3-5:1.

The presence of both the toxoid and the cell-wall antigen will give rise to antibodies against the bacterial cell-wall protein and the complete toxin, so providing a very effective vaccine against scours disease.

The vaccines as described should contain an effective amount of antigenic protein in order to prepare a vaccine useful for effective immunisation of the host. In the case of pig scours a unit dosage of the vaccine suitably contains from 2 ug to 10 ug of toxoid (based on protein composition) per Kg of animal body weight and from 5 ug to 20 ug/Kg of each of the K88 antigens. In the case of whole bacteria the vaccine will preferably contain the equivalent weight of whole cells required to produce the dosage of the purified antigens.

The vaccines may be administered orally, intramuscularly or subcutaneously. In the case of pig scours vaccine, the vaccine may be administered to pregnant sows in two doses 3-4 and 6-8 weeks before farrowing. The vaccine may also be administered to young animals to prevent scouring.

According to a still further aspect of the invention we provide a method for combating a disease caused by a toxin which comprises administering to a human or animal subject an effect dose of a vaccine as described above.

The vaccine as described above, or indeed any vaccine prepared using a toxoid as hereinbefore described, has considerable advantages over previously used vaccines, not only in effectiveness since an unattenuated vaccine is being used, but also in safety, since the bacteria used during fermentation of the vaccine may be non-pathogenic, and there is no risk of unmodified toxins being present in the administered vaccine.

The following Examples, which are of a nonlimiting nature, illustrate the production of LT toxoids according to the methods herein described together with fermentation procedures.

The term "ug" is used herein to denote micrograms.

141-436D2

Materials

Dithiothreitol (DTT), spermidine, agarose Type 1, sodium dodecyl sulphate (SDS), 2-mercaptoethanol, phosphoenol pyruvate, folinic acid, diethyl pyrocarbonate, tRNA (*Escherichia coli*), amino acids, ATP, CTP, GTP, UTP, cAMP, thymidine, nicotinamide, $NAD^+$, creatine phosphate, bovine serum albumin, creatine phosphokinase, brilliant blue R250, bromophenol blue, RNase A and pyruvate kinase were from Sigma (London) Chemical Co.

d ATP, d CTP, d GTP, d TTP, dd ATP, dd CTP, dd GTP and dd TTP were from P-L Biochemicals, Division of Pharmacia, Inc.

T4 DNA ligase and the restriction enzymes *Bam* H1, *Eco* R1, *Hinc* II, *Hind* III, *Pst* 1, *Sau* 3A, *Sma* 1, *Sst* II and *Xba* 1 were from Bethesda Research Laboratories (UK) Ltd. Calf intestinal alkaline phosphatase and T4 polynucleotide kinase and Klenow Large Fragment DNA polymerase I were from The Boehringer Corporation (London) Ltd.

Agarose A-5m was from Bio-Rad Laboratories Ltd. Acrylamide and N,N'-methylenebisacrylamide were from BDH Chemicals Ltd.

($\alpha$-$^{32}P$) ATP, ($^3H$) cAMP and ($^{35}S$) methionine were from Amersham International plc.

($\alpha$-thio-$^{35}S$) d ATP was from New England Nuclear, W. Germany.

Partisil 10-SAX bondapak and spherisorb 5.O.D.S. were from R.O.D. Scientific Merseyside.

All solutions were made up in high quality HPLC grade water. The pH of solutions was adjusted by the addition of appropriate acid or alkali as required.

Bacterial Strains and Vectors

*E. coli*, NCIB 11932, containing the plasmid P307, was used as a source of the LTA and LTB genes.

Plasmid P307 has a molecular weight of about 60 x $10^6$ and is known to encode active LT (Dallas W.S., D.M. Gill and S. Falkow (1979), J. Bacteriol. 139 850 - 858).

The E. coli strain used as the recipient for cloning with the hybrid plasmids was NCIB 11933 (pro$^-$, leu$^-$, thi$^-$, lac Y$^-$, hsd R$^-$, end A$^-$, rec A$^-$, rps L20, ara-14, gal K2, xyl-5, mtl-1, sup E44).

The vector used in this work was pAT153, a derivative of pBR322. The derivation of pAT153 has been described elsewhere (Twigg, A.J. and D. Sherratt (1980) Nature 283, 216-218).

The E.coli strain used for M13 phage manipulations was E.coli K12 (JM103) (lac$^-$, pro$^-$, thi$^-$, str A$^-$, end A$^-$, sbc B15, hsd R4, sup E, ftra D36, pro AB lac I$^q$, Z$^-$M15) and this was obtained from Bethseda Research Laboratories. M13mp10 RF DNA was obtained from Amersham International plc.

Results

The results obtained are illustrated by reference to the accompanying drawings, in which:-

Figure 1 shows the nucleotide sequence for the native LTA gene.

Figure 2 shows the nucleotide sequence of the LTA gene of the site directed mutant SDMI.

Figure 3 shows the plasmid pAT153. This plasmid confers resistance to ampicillin and tetracycline and has a number of unique restriction enzyme recognition sequences suitable for cloning.

Figure 4 shows the plasmid construct GpELTB-10. This plasmid confers ampicillin resistance and produces the LTB protein subunit.

Figure 5 shows the plasmid construct GpELTA-6. This plasmid confers resistance to tetracycline and produces the LTA subunit.

Figure 6 shows the plasmid construct GpENT-2. This plasmid confers resistance to tetracycline and produces both LTA and LTB subunits.

Figure 7 shows

a) The nucleotide sequence of LTA gene in the region of the XbaI site (TCTAGA).

b) The protein sequence corresponding to the nucleotide sequence in a).

c) The double stranded synthetic oligonucleotide to be inserted into the LTA gene.

d) The nucleotide sequence in the LTA gene after insertion of the oligonucleotide into the XbaI site, the XbaI site (TCTAGA) being replaced by two unrecognised sequences (TCTAGT and ACTAGA) with the formation of a new SstII site (CGGCGG).

e) The protein sequence corresponding to nucleotide sequence in d).

Figure 8 shows the plasmid construct GpENT-2s5 which confers resistance to tetracycline and produces both the LTB subunit and the modified LTA subunit containing the oligonucleotide d(CTAGTCCGCC

Example 1

Production of an LT-Toxoid by Chemical Mutagenesis

A) Cloning of the LTB Gene into pAT153

i) Large Scale Plasmid Preparation of pAT153

E. coli NCIB 11933 (containing pAT153) was grown in 1L of L-broth (1% tryptone, 0.5% yeast extract, 0.5% NaCl) with shaking at 37°C. When an optical density $(OD)_{550}$ of 0.6 was attained chloramphenicol was added to give a final concentration of 170 ug/ml. After a further 17 hr incubation at 37°C the culture was centrifuged at 10,000 g for 10 min. The bacterial pellet was resuspended in 30 ml of 25% (w/v) sucrose in 50mM Tris-HCl, 1mM EDTA, pH 8.0. This was put on ice and 6.0 ml of lysozyme solution (5 mg/ml in 250mM Tris-HCl, pH 8.0) was added with gentle mixing. After 5 min incubation on ice, 9.0 ml of 250mM EDTA, pH 8.0, was added again with gentle mixing. After incubation for 5 min on ice, 35 ml of 0.2% (v/v) Triton X-100 in 50mM Tris-HCl, 62.5mM EDTA, pH 8.0, was added again with gentle mixing. After incubation for 15 min the lysate was centrifuged at 50,000 g for 20 min; the resulting supernatant was collected and retained. To this supernatant was added 1/10th volume 5M NaCl and 10% (w/v) polyethylene glycol (4000) to precipitate the DNA. After standing for 4 hr at 4°C the solution was centrifuged at 10,000 g for 10 min. The pellet was resuspended in 8.0 ml 50mM Tris-HCl, 1mM EDTA, pH 8.0. To this was added 7.6 g solid CsCl and 0.3 ml of ethidium bromide solution (10 mg/ml). The mixture was dispensed into two 5 ml quick seal tubes and centrifuged at 250,000 g for 15 hr in a high-speed vertical rotor at 15°. The plasmid band was observed by illumination with long-wave ultra-violet light and removed using needle and syringe. The solution was extracted with isoamyl alcohol until colourless. The solution was then heated at 65° for 15 min (to destroy residual nucleases) and dialysed against 2 x 100 volumes of 10mM-Tris-HCl, 10mM NaCl, 0.1mM EDTA, pH 8.0 at 4°C. After 4hr this dialysis was repeated.

Plasmid concentration was determined by measuring the $OD_{260}$ of the solution. An $OD_{260}$ value of 1.0 in a path length of 1 cm is equivalent to 50 ug/ml of DNA.

ii) Digestion of pAT153 with Hind III

5 ug of pAT153 was dissolved in 30 ul of reaction buffer (final concentration 20mM Tris-HCl, 10mM $MgCl_2$, 1mM DTT, 50mM NaCl, pH7.5). The enzyme Hind III (5 units/ul) was added to the reaction mixture (1 unit/ug of DNA) and this was incubated at 37°C.

The course of the reaction was followed using agarose gel electrophoresis as follows.

0.7% agarose slab gels (25 x 20 x 0.4 cm) were prepared in running bufer (40mM-Tris acetate, 2mM EDTA, pH7.8). 5 ul of running dye (30% glycerol (v/v), 0.1% (w/v) bromophenol blue in running buffer) was added to the DNA sample (0.2 g) and made up to a total volume of 25ul with running buffer. The sample was loaded onto the gel which was run for 4 hr at 100 mA. The gel was stained for 30 min in a solution of ethidium bromide (0.5 ug/ml), viewed on a transilluminator (model C63, U.V. Products) and photographed on Type 57 landfilm on a Polaroid MP3 land camera fitted with a #15 and red filters (Wratten)

The digestion mixture was monitored every 2hr as described until digestion of pAT153 by Hind III was complete.

iii) Dephosphorylation of digested pAT153

2 ug of digested pAT153 (12ul) were withdawn and 1 unit (1ul) of calf intestinal alkaline phosphatase was added and incubation continued at 37°C for 60 min. The mixture was treated with an equal volume of phenol containing 0.1% (w/v) 8-hydroxyquinoline (all stored under equilibration buffer consisting of 10mM Tris-HCl, 0.2% 2-mercaptoethanol, pH 8.0). The aqueous phase was removed and extracted with an equal volume of chloroform: isoamyl alcohol (24:1) and finally with an equal volume of chloroform.

The DNA was precipitated from aqueous solution by the following procedure. To the DNA solution was added 1/10th volume of 3M sodium acetate pH 5.5 followed by 2 volumes of absolute ethanol. The solution was mixed well and placed in a dry-ice/ethanol bath for 30 min. After centrifugation at 13,000 g for 3 min the supernatant was discarded and the DNA pellet dried under vacuum.

The precipitate was dissolved in 2 ul of 10mM Tris-HCl, 10mM NaCl, 0.1mM EDTA, pH8.0. This DNA is the vector molecule into which the insert containing the LTB gene will be cloned.

iv) Large scale plasmid preparation of P307

The method used was as described in A.i) except that E.coli NCIB 11932 containing P307 was substituted for E.coli NCIB 11933 containing pAT153. Furthermore, the addition of chloramphenicol to the 1L culture was omitted; the culture being harvested at an $OD_{550}$ of 2.0.

v) Digestion of P307 with Hind III

The method was as described in A ii) except that 10 ug P307 DNA was substituted for 5ug pAT153. The digested DNA was extracted with phenol and precipitated in ethanol as described in A iii).

vi) Ligation of pAT153 and P307

0.5 ug of cut vector from A iii) and 1.5 ug of cut insert from A v) were added to 100 ul of ligation mixture containing 50mM Tris-HCl, 10mM $MgCl_2$, 0.1mM ATP, 20mM, DTT, 500 ug/ml bovine serum albumin, pH 7.8. The ligation was started by the addition of 1 unit of T4 DNA ligase (1 ul) and incubated for 12 hr at 12°.

vii) Bacterial Transformation

The DNA mixture derived as described in steps (A i-vi) was used to transform NCIB 11933.

An overnight culture of E. coli NCIB 11933 was diluted a hundred-fold into fresh L-broth (30 ml). This culture was grown with vigorous shaking at 37°C. At an $OD_{550}$ of 0.4 - 0.5 the culture was cooled in an ice bath for 5 min. The culture was centrifuged at 6,000 g for 5 min and the pellet washed with 20 ml of ice cold 0.15M NaCl and again centrifuged at 6,000 g for 5 min. The pellet was resuspended in 15 ml of ice cold solution of $CaCl_2$ (50mM). After 30 min standing on ice the cells were again pelleted by centrifugation and resuspended in 3 ml of the $CaCl_2$

solution. To 0.2 ml of this cell suspension was added 100 ul of the DNA mixture (1 ug in 50mM $CaCl_2$). Following a further 30 min incubation on ice the suspension was transferred to a waterbath at 43° and incubated for 2 min. The suspension was returned to the ice bath for 20 min and 4.0 ml of L-broth was added. The cells were incubated at 37° for 60 min and plated, at serial dilution, onto selective media.

The selective medium was L-agar (L-broth containing 1.5% w/v agar) containing ampicillin at 100 ug/ml.

viii) Analysis of Hybrid Plasmids

A small-scale plasmid preparation was made of each of the ampicillin resistant transformants as follows:-

1.0 ml of culture was grown overnight at 37°C in L-broth. The cells were pelleted by centrifugation at 13,000 g for 2 min. The pellet was resuspended in 25 ul of 8% (w/v) sucrose, 5% (v/v) Triton X-100 in 50mM Tris-HCl, 50mM EDTA, pH 8.0, and mixed well. 2ul of lysozyme solution (10 mg/ml in 250 mM Tris-HCl, pH 8.0) was added and the tube placed in a boiling water bath. After 40 sec the tubes were centrifuged at 13,000 g for 10 min. The supernatant (20 ul) was removed and mixed with an equal volume of isopropanol and placed in a dry-ice/ethanol bath. After 10 min the mixture was centrifuged at 13,000 g for 10 min.

The supernatant was discarded and the pellet redissolved in 20 ul 10mM Tris-HCl, 50mM NaCl, 0.1mM EDTA. The DNA was precipitated from the solution using the ethanol precipitation procedure described in A iii). The pellet was resuspended in 20ul of 40mM Tris-acetate, 2mM EDTA, pH 7.8) and analysed by agarose gel electrophoresis as in Aii).

Using this analysis a number of transformants were identified which contained hybrid plasmids i.e. a combination of pAT153 vector plus an inserted fragment derived from P307.

ix) Assay for LTB Activity

The isolated transformants identified in A viii) were each grown in 500 ml of L-broth to late exponential phase and harvested by centrifugation at 10,000 g for 5 min. The pellet was washed in 100 ml 50mM Tris-HCl, pH 7.8 and again centrifuged at 10,000 g for 5 min. The pellet was resuspended in 10 ml of TEAN. The suspension was sonicated, with cooling in an ice-bath, sufficient to rupture 85-95% of the total cells. The cell debris was pelleted by centrifugation at 30,000 g for 1 hr and the supernatant was stored at 4°C until required.

Each extract was assayed for LTB activity using a ganglioside immunosorbent assay ($G_{M1}$-ELISA) procedure (Svennerholm A.M. and J. Holmgren (1978) Curr. Microbial. 1, 19-23). One such extract was found to contain a high level of LTB activity. This activity was encoded by the NCIB 11933 transformant containing the hybrid plasmid GpELTB-10.

B) Cloning of the LTA Gene into pAT153

The LTA gene was cloned into pAT153 in substantial accordance with the teaching of Example 1A i)-ix).

i) Digestion of pAT153 with EcoRl and HincII

5 ug of pAT153 was digested to completion with EcoRl as described in A ii) except that the final concentration of the reaction buffer was 20mM Tris-HCl, 10mM $MgCl_2$, 1mM DTT, 100mM NaCl, pH7.5. The DNA was precipitated from ethanol as in Aiii) and resuspended in 30 ul buffer containing 20mM Tris-HCl, 10mM $MgCl_2$, 1mM DTT, 50 mM NaCl, pH7.5. To this was added 0.1 unit of Hinc II to give partial digestion of the above DNA. This was achieved by monitoring the reaction at 30 min intervals by agarose gel electrophoresis to determine the time required that resulted in optimum production of molecules only cut in one position by Hinc II. The DNA was extracted with phenol and precipitated from ethanol as described in Aiii).

ii) Digestion of P307 with EcoRl and Hpal

10ug of P307 was digested to completion with Hpal as described in Aii) except that the final concentration of the reaction buffer was 20mM Tris-HCl, 10mM $MgCl_2$, 1mM DTT, pH 7.5. After digestion the DNA was precipitated from ethanol as in Aiii) and resuspended in 30 ul buffer containing 20mM Tris-HCl, 10mM $MgCl_2$, 1mM DTT, 100mM NaCl, pH 7.5. To this was added 10 units of EcoRI and the digestion monitored by agarose gel electrophoresis to completion (see A ii)). The DNA was extracted with phenol and precipitated from ethanol as described in A iii).

iii) Ligation of pAT153 and P307

0.5 ug of cut vector pAT153 from B(i) and 1.5 ug of cut insert from B ii) were added to 100 ul of ligation buffer and ligated according to the procedure described in A iv).

iv) Bacterial Transformation

The DNA mixture derived as described in Bi)-iii) was used to transform NCIB 11933. The transformatior procedure was as described in A vii) except that the selective medium was L-agar containing tetracycline at 25 ug/ml. The successful tetracycline resistant transformants were each plated onto L-agar containing ampicillin at 100 ug/ml and those found to be sensitive to ampicillin were tested as follows.

v) Analysis of Hybrid Plasmids

A small scale plasmid preparation was made of each of the tetracycline resistant, ampicillin sensitive transformants as described in A viii).

A number of transformants were isolated which contained hybrid plasmids; i.e. a combination of pAT153 vector plus an inserted fragment from P307.

vi) Assay of LTA Activity

Cell-free extracts were made of each of the transformants identified in B v) according to the method given in A ix).

Each extract was assayed for LTA activity by measuring toxin stimulation of adenyl cyclase (Tait, R.M., B.R. Booth and P.A. Lambert (1980) Biochem. Biophys. Res. Comun. 96 1024-1031). One such extract was found to contain a high level of LTA activity. This activity was encoded by the NCIB 11933 transformant containing the hybrid plasmid GpELTA-6.

A large scale plasmid preparation of GpELTA-6 was made as described in A i)

C) Isolation of a derivative of Gp ELTA-6 encoding a non-active LTA protein.

i) Chemical Mutagensis of GpELTA-6

The reaction mixture (400 ul) contained 6 ug of plasmid GpELTA-6 (from B vi) in 100mM sodium phosphate, 1mM EDTA, 400mM hydroxylamine, pH 6.0. The mixture was incubated at 65°C for 4 hr under mineral oil, followed by dialysis against 10 volumes of 50mM $CaCl_2$ at 4°C. After 4hr this dialysis was repeated.

ii) Transformation with Mutagenised GpELTA-6

Following hydroxylamine mutagenesis the GpELTA-6 DNA was used to transform a culture of NCIB 11933 as described in A vii). The selective medium was L-agar containing 25 ug/ml tetracycline.

iii) Assay of LTA Activity

Cell free extracts were made of each of the transformants isolated in C ii) and assayed for LTA activity as described in B vi). A number of transformants were isolated that did not produce active LTA. These mutants were analysed further as follows.

iv) Analysis of Mutants by in Vitro Transcription/ Translation

Large scale plasmid preparations were made from each of the mutants having no LTA activity (from C iii)) as described in A i). The protein products of the plasmids were analysed using an *in vitro* transcription /translation system (Yang H.L., Heller K., Geller M and Zubay G. (1979) Proc. Natl. Acad. Sci. USA 75 3304-3308) followed by SDS polyacrylamide (15%) gel electrophoresis (Laemmli, U.K. (1970) Nature 227, 680-685). The gel was dried and exposed to a film of Kodak AR5 overnight followed by its development.

From the results three mutants were chosen for further analysis. The plasmids from these mutants were each found to encode a protein from the LTA gene that was indistinguishable in size from the native LTA protein. The mutants in NCIB 11933 contained the plasmids designated GpELTA-6ml-6m2 and -6m3.

v) Determination of Nucleotide Sequence for the LTA Protein and the Three Mutants

The LTA gene in GpELTA-6 and the three mutated plasmids GpELTA-6ml, GpELTA-6m2 and GpELTA-6m3 were sequenced using either the dideoxynucleotide method (Sanger, F., S. Nicklen and A.R. Coulson (1977) Proc. Natl. Acad. Sci USA 74 5463-5467) or the chemical cleavage technique (Maxam, A.M. and W. Gilbert. (1977) Proc. Natl. Acad. Sci USA 74 560- 564). The sequences and positions of the mutations are shown in Fig. 1.

D) Construction of Hybrid Plasmid Encoding Whole Toxin/Toxoid

The hybrid plasmids encoding whole toxin/toxoids were constructed in accordance with the teaching of Example A i)-ix).

i) Digestion of GpELTA-6 with EcoRl

The plasmid GpELTA-6 (5 ug) (from B iv)) was digested with EcoRl to completion as described in A ii) except that the final concentration of the reaction buffer was 20mM Tris-HCl, 10mM $MgCl_2$, 1mM DTT, 100mM NaCl, pH7.5. The digested DNA was dephosphorylated, extracted with phenol and precipitated from ethanol as described in A iii).

ii) Digestion of GpELTA-6ml, GpELTA-6m2 and GpELTA-6m3 with EcoRl.

A large scale plasmid preparation of each of the plasmids GpELTA-6ml, GpELTA-6m2 and GpELTA-6m3 (from C iv)) was prepared as decribed in A i). Each plasmid was digested with EcoRl, dephosphorylated, extracted with phenol and precipitated from ethanol as described in D i).

iii) Digestion of GpELTB-10 with EcoRl

A large scale plasmid preparation of GpELTB-10 (from A ix)) was made as described in A i). 5ug of this plasmid was digested with EcoRl to completion, extracted with phenol and precipitated from ethanol as described in D i).

iv) Ligation of LTB coding fragment with GpELTA-6 and its Derivatives

0.5ug of cut vector, GpELTA-6 from D i) or one of each of the three GpELTA-6 mutants from D ii) and 1.5ug of cut insert from D iii) were added to 100ul of ligation buffer and ligated according to the procedure described in A vi).

v) Bacterial Transformation

Each of the four ligation mixtures from D iv) were used to transform NCIB 11932 as described in A vii). The selective medium was L-agar containing tetracycline at 25 ug/ml.

vi) Analysis of Hybrid Plasmids

A small-scale plasmid preparation of each of the tetracycline resistant transformants (from the four ligation mixtures) was made as described in A viii).

Using this analysis a number of transformants were identified which contained a hybrid plasmid; i.e. a combination of GpELTA-6 (or one of its three mutant derivatives) and an insert fragment from GpELTB-10, known to encode the LTB gene.

vii) Assay for LTB Activity

As the cloning of the LTB encoding fragment could be in either of two orientations it was necessary to determine that LTB is expressed when cloned into the LTA encoding plasmid.

The isolated transformants identified in D vi) were each assayed for LTB activity as described in A ix).

A transformant from the GpELTA-6/GpELTB-10 plasmid ligation mixture that expressed a high level of activity of LTB was selected. The hybrid plasmid was termed GpENT-2. Similarly, a transformant was isolated from each of the other ligation mixtures that expressed a high level of activity of LTB. These hybrid plasmids were termed GpENT-2m1, GpENT-2m2 and GpENT-2m3.

viii) Purification of LT Toxin/Toxoid

Cell-free extracts were prepared (as described in A ix) from bacterial strains containing GpENT-2, GpENT-2m1, GpENT-2m2 and GpENT-2m3. Each cell-free extract (8 ml) was applied onto a column of agarose A-5m (35x1.4 cm) equilibrated with TEAN at 4°C.

TEAN was applied to the column until the $OD_{280}$ of the effluent had returned to baseline level. The LT, which at this stage was bound to the column, was eluted by the application of 0.2 M D-(+)-galactose in TEAN. The eluted fraction was found to be highly purified for LT (usually about 95%) and was stored at 4°C until further analysis.

The purified toxin, from GpENT-2 and the three toxoids from GpENT-2m1, GpENT-2m2 and GpENT-2m3 were collected and their gross structure was studied further.

ix) Structure of Toxin/Toxoids

The purified toxin/toxoids from D viii) were analysed by SDS-polyacrylamide gel electrophoresis (15%) (Laemmli U.K. (1970) Nature 227 680-685). The results show that for the toxin and each of the three toxoids there are two protein components corresponding to the size of LTA and LTB. Furthermore analysis by densitometry tracing shows that the LTA to LTB ratio is the same for the toxin and each of the toxoids.

The results show that each of the mutated LTA molecules is capable of associating with the LTB molecules in a manner indistinguishable from the native LTA.

This result has further been confirmed by immunoprecipitation of toxin/toxoids from cell-free extracts of strains containing GpENT-2, GpENT-2ml, GpENT-2m2 and GpENT-2m3 using LTB specific antisera.

x) Testing in vivo Activity of the Toxin/toxoids

Cell-free extracts of bacterial strains containing GpENT-2, GpENT-2ml, GpENT-2m2, GpENT-2m3 were tested in the rabbit ileal loop test. (Evans, D.G., D.J. Evans, Jr. and n.F. Pierce (1973) Infect. Immun. 7 873-880).

It was demonstrated that none of the three mutants had appreciable activity when compared with the natural toxin. In fact it has not been possible to detect toxic activity of any kind from the extract of GpENT-2ml even when used at a 2000 fold excess of the toxin level required to produce a positive response.

Using the above technique eight further toxoids were prepared, namely 4-11 as shown in the Table hereinbefore. These too have been shown to lack the toxicity of the natural toxic component.

## Example 2

### Production of an LT-toxoid by Oligonucleotide Insertion

### Inactivation of LTA gene by Oligonucleotide Insertion

The presence of the Xbal site in the LTA gene (Fig.4) allows for the mutation of the gene by the insertion of a synthethic oligonucleotide at this site. The nucleotide sequence and corresponding protein sequence in this region of LTA is shown in Fig. 7. A synthetic oligonucleotide adaptor, 12 nucleotides in length, is shown in Fig. 7 and in this same figure it can be seen how the insertion of this oligonucleotide results in the:-

1. changed gene sequence
2. correspondingly altered protein sequence
3. loss of the Xbal site
4. acquisition of an SstII site.

A) Synthesis of the Oligonucleotide d (CTAGTCCGCGGA) (i) Column Drying

The polydimethyl-acrylamide/Kieselguhr support functionalised with 5'-dimethoxytrityl-N-benzoyl-2'-deoxyadenosine (53.7 mg), in a small glass column was treated with pyridine and stirred briefly to remove any air bubbles. The resin was washed with pyridine (10 min; flow rate ca 1.0ml/min). The pyridine flow was stopped and phenyl isocyanate (0.1ml) in pyridine (0.9ml) was injected over a period of 5 min and the column then left for 4 min.

(ii) Deprotection and Coupling Reactions

The polydimethylacrylamide/Kieselguhr support functionalised as above was washed successively with pyridine (5 min; flow rate 1 ml/min) and 1,2-dicholoroethane (3min). The dimethoxytrityl group was removed by washing with 10% trichloroacetic acid in 1,2-dichloroethane (3 min). The resin was then washed successvely with dimethylformamide (5 min) and pyridine (5 min). The flow was stopped and 5'-dimethoxytrityl-N-isobutyryl-2'-deoxyguanosine 3'-(2-chlorophenyl)phosphate triethyl ammonium salt (37.5mg,) in pyridine (0.4ml) containing 1-(mesitylene-sulphonyl)3-nitro-1, 2, 4-triazole (45 mg); was injected into the column over a period of 3 min and then left for 45 min at RT.

The resin was washed successively with pyridine (5 min) and then 1,2-dichloroethane (3 min). The dimethoxytrityl group was removed as before by treatment with 10% trichloroacetic acid in 1,2-dichloroethane (3 min). The resin was then washed successively with dimethyflormamide (5 min) and pyridine (5 min). The next nucleotide (40 umol) in pyridine 0.4ml containing 1-(mesitylene-sulphonyl)-3-nitro-1,2,4-triazole (45mg.) was injected via the septum port over a period of 3 min and left at RT (45 min).

This procedure was repeated using the appropriate protected nucleotide, namely either
5'-dimethoxytrityl-N-isobutyryl-2'-doexyguanosine-3'-(2-chlorophenyl) phosphate triethyl ammonium salt or
5'-dimethoxytrityl-N-benzoyl-2'-deoxycytidine-3'-(2-chlorophenyl) phosphate triethyl ammonium salt or
5'-dimethoxytrityl-N-benzoyl-2'-deoxyadenosine-3'-(2-chlorophenyl) phosphate triethylammonium salt or
5'-dimethoxytritylthymidine-3'-(2-chlorophenyl)phosphate triethylammonium salt
until the desired sequence had been assembled.

(iii) Deprotection

When the assembly has been completed the resin was washed with pyridine (10 min) and 1,2-dichloroethane (5 min). The resin was removed from the column and washed with ether (5 x 10 ml).

The resin was treated with a solution of syn-2-nitrobenzaldoxime (140 mg) in dioxan-water (1:1) (2 ml) containing 1,1,3,3-tetramethylguanidine (0.1 ml). After 18 hr the mixture was filtered, the residue washed with water (2 ml) and the filtrate evaporated to dryness. The residue was dissolved in concentrated ammonia solution (5 ml) and heated at 60°C. After 3 hr, the solution was evaporated to dryness and the residue treated with acetic acid-water (4:1) (5 ml). After 30 min the solution was evaporated to dryness and the residue dissolved in water (3 ml) and extracted with diethylether (5 x 10 ml). The aqueous liquors were evaporated under reduced pressure and the residue dissolved in water (1.2 ml).

(iv) Purification

The crude material was purified by anion exchange HPLC on partisil 10-SAX at 40°C using (A) 0.001M potassium dihydrogen phosphate in formamide-water (6:4) pH 6.3 and a gradient of 0.07% (B) 0.03 potassium dihydrogen phosphate in formamide-water (6:4) pH 6.3 over a period of 45 min.

In a typical preparative run a sample (150 ul) of crude oligonucleotide solution was injected. A gradient of 0.70% eluent B was run over a period of 45 min. The eluate after ca 40 min containing the desired material (as indicated by a UV detector set at 270 nm) was collected. The remaining material was purified in a similar manner. The purified material was combined (15ml) and desalted in two portions by passage through a Biogel P2 column (void volume 60 ml) eluting with ethanol-water (1:4). The eluate at the void volume was combined (40 ml) and evaporated to dryness.

The residue was dissolved in water (0.5ml). Yield of d(CTAGTCCGCGGA) = 9 O.D.(260 nm).

This material was analysed by reversed phase HPLC on bondapak and spherisorb 5.O.D.S. columns using a 0.1M ammonium acetate at pH 6.3 and a gradient of 0.10% 0.1M ammonium acetate in acetonitrile-water (4:1) at pH 6.3 over a period of 25 min. This protocol was not used preparatively as further purification proved unnecessary.

B) Insertion of Oligonucleotide Adaptor into the Xbal Site GpELTA-6

i) Phosphorylation of Oligonucleotide Adaptor

A 1 ug sample of the synthetic oligonucleotide d(CTAGTCCGCGGA) from Example 2 A in a total volume of 5 ul 50mm Tris-HCl, pH 7.5 was heated to 65°C for 5 min. When cooled the DNA solution was added to 50 ul of reaction buffer containing 50mM Tris-Hcl, 5mM DTT, 0.1mM spermidine, 0.1mM EDTA, 4 mM

ATP, pH 7.5. 10 units of T4 polynucleotide kinase (10 units/ul) was added to the reaction mixture which was incubated at 37°C for 30 min. The mixture was extracted with phenol as described in Example 1 A iii). 1 ul of a solution of tRNA (*E. coli*) (10 mg/ml) was added to the mixture and the DNA precipitated from ethanol as described in Example a. A iii). The precipitate was dissolved in 5 ul 10mM Tris-HCl, 0.1mM EDTA, pH 7.5.

ii) Digestion of GpELTA-6 with Xbal

5 ug of GpELTA-6 was digested to completion with Xbal as described in Example B (ii) except that the final concentration of the reaction buffer was 20mM Tris-HCl, 10mM Mg $Cl_2$, lmM DTT, 100mM Nacl, pH 7.5. The DNA was extracted with phenol and precipitated from ethanol as described in Example 1 A iii).

iii) Ligation of GpELTA-6 and the Synthethic Oligonucleotide

100 ng (5 ul) of the phosphorylated oligonucleotide from B i) and 1 ug of the digested Gp ELTA-6 from B ii) were ligated in 100 ul of reaction buffer as described in Example 1 A vi). The mixture was extracted with phenol and precipitated from ethanol as described in Example 1 A iii). The precipitate was resuspended in 5 ul of 10mM Tris-HCl, 0.1mM EDTA, pH 7.5.

iv) Digestion of GpELTA-6/Oligonucleotide Hybrid with Sst II

The DNA from B iii) was digested to completion with Sst II as described in Example 1A ii). The mixture was loaded onto a Sephadex G50 column (0.9 x 15 cm) equilibrated in 50mM Tris-HCl, 10mM Mg $Cl_2$, 20mM DTT, 0.1mM ATP pH 7.8 and the DNA eluting in the void volume was collected in 100 ul. 5 ul of the bovine serum albumin solution (10 mg/ml)was added followed by 1 unit of T4 DNA ligase (1 ul) and incubated for 12 hr at 12°C.

v) Bacterial Transformation

The DNA mixture from B iv) was used to transform NCIB 11933. The transformation procedure was as described in Example 1 A vii) except that the selective medium was L-agar containing tetracycline at 25 ug/ml.

vi) Analysis of Hybrid Plasmids

Small scale plasmid preparations were made of the tetracycline resistant transformants from B v) as described in Example 1 A viii). However, following ethanol preciptation the DNA was resuspended in 60 ul of 20mM Tris-HCl, 10mM Mg $Cl_2$, 1mM DTT, 50mM NaCl, pH 7.5. This DNA solution was divided into three aliquots; one was treated with 1 unit Xba 1 (1 unit/ul) the second with 1 unit Sst II (lunit/ul) while the third remained untreated. These DNA solutions were analysed by agarose gel electrophoresis as described in Example 1 A ii). A transformant was identified that contained hybrid plasmid which had no Xba 1 site but a single Sst II in its place. This plasmid was GpELTA-6s5 and had the oligonucleotide adaptor d(CTAGTCCGCGGA) inserted into the unique Xbal site in the LTA gene of plasmid GpELTA-6.

C) Analysis of Toxoid Encoded by the GpELTA-6s5 Plasmid

i) Assay of LTA Activity

A cell-free extract was made of the transformant containing Gp ELTA-6s5 as described in Example 1 A ix). The extract was assayed for LTA activity as described in Example 1 B vi). The extract was found to lack LTA activity.

A large scale preparation of GpELTA-6s5 was made as described in Example 1 A i).

ii) Analysis of GpELTA-6s5 by in Vitro Transcription/Translation

GpELTA-6s5 was used as a template for the *in vitro* transcription/translation process described in Example 1 C iv). Following SDS polyacrylamide gel electrophoresis and autoradiography it was found that the LTA gene with the oligonucleotide insert in GpELTA-6s5 encoded a protein that was larger than LTA by about a molecular weight of 500. This was accounted for by the extra 4 amino acids coded within the inserted oligonucleotide.

iii) Construction of a Toxoid Encoding Plasmid

The method used was as described in Example 1 D i), iii)-vii) except that GpELTA-6s5 was substituted for GpELTA-6. A hybrid plasmid was identified that contained both the LTB gene and the LTA gene with the oligucleotide insert. This plasmid was GpENT-2s5.

iv) Determination of in Vivo Activity of the Toxoid

A cell-free extract of NCIB 11933 (containing GpENT-2s5) made as described in Example 1 A ix) was tested in the rabbit ileal-loop test as in Example 1 D x).

No measurable toxic activity was observed even when used at a 2000 fold excess of the toxin-level required to produce a positive response.

The bacteria containing the LT toxoid products of Examples 1 and 2 may be freeze dried and stored.

Example 3

Production of an LT-Toxoid by Site Directed Oligonucleotide Mutagenesis

A mutant LTA can be constructed by changing the codon TCC encoding a serine at amino acid position 79 for TTC which encodes a phenylalanine.

A) Construction of an M13mp10/LTA Hybrid Phage DNA

i) Digestion of M13mp10 with EcoR1 and Xbal

5 ug of M13mp10 was digested to completion with EcoR1 and Xba1 as described in Example 1 A ii) except that the final concentration of reaction buffer was 20m Tris-HCl, 10 mM $MgCl_2$, 1mM DTT, 100mM NaCl, pH 7.5. After digestion the DNA was extracted with phenol and precipitated from ethanol as in Example 1 A iii).

ii) Digestion of GpELTA-6 with EcoR1 and Xba1

5 ug of GpELTA-6 from Example 1 B iv) was digested to completion with EcoR1 and Xba1 as described above in A i).

iii) Ligation of LTA Fragment into M13mp10

0.5 ug of cut vector from A i) and 0.5 ug of insert DNA from A ii) were added to 100 ul of ligation buffer and ligated according to the procedure described in Example 1 A vi).

iv) Bacterial Transformation

The ligation mixture from A iii) was used to transform E.coli as described in Example 1 A vii) except that strain JM103 was used as the competent host. The selective medium was L-agar contained 0.3mM isopropyl-β-D-thiogalactopyranoside and 0.03% (w/v) 5-bromo-4-chloro-3-indolyl-β-D;galactoside. After plating the transformants, the plates were overlayed with 3ml of L-broth containing 0.7% (w/v) agar (previously melted and stored at 50°) to which had been added 10 ul of an exponentially growing culture of JM103 ($OD_{550}$ = 0.7). A transformation event can be observed by the presence of a viral plaque on the JM103 'lawn'. These plaques are of two types, blue and colourless, dependent on the reaction with the selective medium constituents.

v) Analysis of Hybrid/Phage Constructs

A small-scale preparation of extracellular single stranded phage DNA of each colourless plaque was made as follows. The plaque was picked from the plate with a sterile toothpick and added to an exponentially growing culture of JM103 in L-broth (2ml) at an $OD_{550}$ of approx 0.2. This culture was grown at 37° for 6 - 7 hr. 1.5ml of culture was pelleted by centrifugation at 10,000g for 5 min. A 20 ul sample of supernatant was mixed with 4 ul of running dye and analysed by agarose gel electrophoresis as described in Example 1 A ii).

Using this analysis a number of plaques were identified which contained hybrid phage; i.e. a combination of M13mp10 and the area of the LTA gene between the EcoR1 and Xba1 restriction sites. One such transformant was designated as M13mp10-LTA. The nucleotide sequence of the LTA fragment of this hybrid was confirmed by the dideoxynucleotide method described in Example 1 C v).

vi) Large Scale Preparation of Extracellular Single Stranded Phage DNA of M13mp10-LTA

A colourless plaque of M13mp10-LTA was picked from a plate using a sterile toothpick and added to 10ml of L-broth. 10 ul of exponentially growing JM103 ($OD_{550}$ = 0.7) was added and the culture incubated at 37° for 8 - 12 hr. 1.5ml of culture was pelleted by centrifugation at 10,000g for 10 min. The top 0.8ml of supernatant was removed and to this was added 200 ul of a solution containing 20% (w/v) polyethylene glycol, 2.5M NaCl. This mixture was incubated at 20° for 15 min followed by centrifugation at 10,000g for 5 min. The supernatant was discarded and the pellet resuspended in 100 ul of a solution containing 20mM Tris-HCl, 50mM NaCl, 0.1mM EDTA, pH 7.5. The resulting solution was extracted with phenol and the DNA precipitated from ethanol as described in Example 1 A iii). The DNA was resuspended in 25 ul of a solution containing 20mM Tris-HCl, 50mM NaCl, 0.1mM EDTA, pH 7.5.

B) Mutagenesis

i) Synthesis of the Oligonucleotide

d(TGTTTTCACTTCTCTTAG)

The oligonucleotide primer d (TGTTTTCACTTCTCTTAG) was synthesised, purified and analysed using the methods described in Example 2 A.

ii) Phosphorylation of Oligonucleotide Primer

A 1 ug sample of synthetic primer from B i) was phosphorylated as described in Example 2 B i).

iii) Digestion of Extracellular Single Strand M13mp10-LTA DNA with Sau3A

To eliminate trace levels of double stranded DNA from the material produced in A vi), 5 ug of this DNA was digested to completion with Sau3A as described in Example 1 A ii). After digestion the DNA was extracted with phenol and precipitated from ethanol as in Example 1 A iii).

iv) Annealing of M13mp10-LTA Template and the Synthetic Oligonucleotide Primer

0.5 ug of Sau 3A digested M13mp10-LTA template from B iii) and 10ng of phosphorylated oligonucleotide primer from B ii) were dissolved in 10 ul of a solution containing 20mM Tris-Hcl, 10mM $MgCl_2$, 50mM NaCl, 1mM DTT, pH 7.5 and heated to 55° for 5 min. The reaction mixture was chilled at 23° for 5 min. followed by the addition of 10 ul of a solution containing 20mM Tris-HCl, 10mM $MgCl_2$, 10mM DTT, 1mM dATP, 1mM dCTP, 1mM dGTP, 1mM TTP, 1mM ATP, pH 7.5. 5 units of DNA ligase (10 units/ ul) and 2.5 units of 'Large Fragment' DNA polymerase (5 units/ ul) were added to the mixture which was then incubated at 15° for 16 hr.

v) Bacterial Transformation

The ligation mixture from B iv) was used to transform JM103 as described in A iv).

Transformants were observed as colourless plaques on selective medium.

vi) Analysis of Transformants

Nucleotide sequencing of the extracellular single strand phage DNA from the colourless plaques from B v) was determined as described in A v). A plaque was identified in which the codon for amino acid 79 of the LTA gene was TTC, thus encoding a phenylalanine at this position and not TCC encoding serine as in the native protein. This change had been affected by oligonucleotide induced site directed mutagenesis. The mutant with this codon change was designated M13mp10-SDM1.

C) <u>Construction of Toxoid-Producing Plasmid</u>

i) <u>Preparation of M13mp10-SDM1 RF (Double Strand) DNA</u>

A colourless plaque of M13mp10-SDM1 was picked from a plate using a sterile toothpick and added to 10ml of L-broth. 10 ul of exponentially growing JM103 ($OD_{550}$ = 0.7) was added and the culture incubated at 37° for 7 hr. The cells were harvested by centrifugation at 7,000g for 5 min. and the supernatant discarded. The pellet was resuspended in 70 ul of 20mM Tris-HCl, 50mM NaCl, 0.1mM EDTA, pH 7.5. 15 ul of a fresh lysozyme solution (5mg/ml) was added and the solution incubated for 5 min. at 4°. This was followed by the sequential addition of 30 ul 20mM Tris-HCl, 50mM Nacl, 0.1mM EDTA, pH 7.5 and 1.25 ul of an RNase solution (RNase A 10 mg/ml) in 10mM sodium acetate, pH 7.5 boiled for 2 min.). Following 5 min. incubation at 4° 120 ul of 20mM Tris HCl, 50mM NaCl, 0.1mM EDTA, pH 7.5 containing 2% (v/v) Triton X-100 was added and the incubation continued a further 10 min. The solution was subjected to centrifugation at 30,000g for 30 min. The supernatant (approx. 0.15ml) was removed and extracted with phenol and precipitated from ethanol as described in Example 1 A iii).

ii) <u>Digestion of M13mp10-SDMI RF DNA with Pstl and EcoRl</u>

5 ug of M13mp10-SDMI RF DNA from C i) was digested to completion with <u>Pst</u>l and <u>Eco</u>Rl as described

in Example 1 A ii).

iii) Digestion of pAT153 with Pst1 and EcoR1

5 ug of pAT153 from Example 1 A i) was digested to completion wtih Pst1 and EcoR1 as described in Example 1 A ii).

iv) Ligation of pAT153 and M13mp10-SDMI RF DNA

0.5 ug of cut vector from C iii) and 1.5 ug of cut insert from C ii) were added to 100 ul of ligation mixture and ligated according to the procedure described in Example 1 A vi).

v) Bacterial Transformation

The DNA mixture from C iv) was used to transform NCIB 11933 as described in Example 1 A vii). The selective medium was L-agar containing tetracycline at 25 ug/ml.

vi) Analysis of Hybrid Plasmids

A small scale plasmid preparation was made of each of the tetracycline resistant ampicillin sensitive transformants from C v) and analysed as described in Example 1 A viii). One such transformant, GpELTA-SDMI, was identified as containing the Pst1-EcoR1 fragment from M13mp10-SDMI RF in the Pst1-EcoR1 sites of pAT153. A large scale plasmid preparation of GpELTA-SDMI was made as described in Example 1 A i).

vii) Elimination of the 58bp HindIII fragment from GpENT-2

A large scale plasmid preparation of GpENT-2 from Example 1 D vii) was made as described in Example 1 A i). 5 ug of this plasmid was digested with HindIII to give partial digestion as described in Example 1 B i). 0.5 ug of this cut plasmid was ligated according to the conditions described in Example 1 A iv) and used to transform NCIB11933 as described in Example 1 A vii) on selectve medium containing tetracycline at 25 ug/ml. Following analysis by small scale plasmid preparation as described in Example 1 A viii) a transformant was identified which had lost the 58bp HindIII fragment (and thus the EcoRl site located on it) but was otherwise the same as GpENT-2. This plasmid was designated as GpENT-3. A large-scale plasmid preparation of GpENT-3 was made as described in Example 1 A i).

viii) Digestion of GpENT-3 with EcoRl and Xbal

5 ug of GpENT-3 from C vii) was digested with EcoRl and Xbal as described in Example A i).

ix) Dephosphorylation of Digested GpENT-3

2 ug of digested GpENT-3 from C viii) was dephosphorylated as described in Example 1 A iii).

x) Digestion of GpELTA-SDMI with EcoRl and Xbal

5 ug of GpELTA-SDMI from C vi) was digested with EcoRl and Xbal as described in A i).

xi) Ligation of GpENT-3 and GpELTA-SDMI

0.5 ug of dephosphorylated cut vector GpENT-3 from ix) and 1.5 ug of cut GpELTA-SDMI were ligated according to the method described in Example 1 A vi).

xii) Bacterial Transformation

The ligation mixture from xi) was used to transform NCIB 11933 as described in Example A vii). The selective medium was L-agar containing tetracycline at 25 ug/ml.

xiii) Analysis of Hybrid Plasmids

A small scale plasmid preparation was made of each transformant and analysed as described in Example 1 A viii). A number of transformants were identified which had the same molecular weight as GpENT-3. Nucleotide sequencing by the dideoxynucleotide method described in Example 1 C v) of one such mutant confirmed that the LTA gene had the same sequence as the native gene except that the codon encoding the amino acid at position 79 had been mutated from TCC to TTC corresponding to a change in the amino acid at this position of a serine to a phenylalanine. The plasmid carrying this mutation was designated GpENT-3SDMI.

D) Testing the Site-Directed Mutation Derived Toxoid

i) Assay for LTB Activity

The transformant containing GpENT-3SDMI was tested for LTB activity as described in Example 1 A ix) and was found to produce LTB.

ii) Assay for LTA Activity

The same transformant was tested for LTA activity as described in Example 1 B vi). No LTA activity was observed.

iii) Structure of Toxoid

The toxoid produced by this transformant was purified as described in Example 1 D viii) and analysed by SDS-polyacrylamide gel electrophoresis (15%). (Laemmli, U.K. (1970) Nature 227 680-685). The results confirmed that this mutated, inactive LTA molecule is capable of associating with LTB in a manner indistinguishable from the native LTA.

iv) Testing in vivo Activity of the Toxoid

The toxoid was tested for activity in the rabbit ileal loop test as described in Example 1 D x). No toxic activity was detected using the site-directed mutation-derived toxoid.

Example 4

Fermentation for the production of LT-toxoid

Strain : The strain, a genetically engineered derivative of E.coli NCIB 11933 was constructed as described in the previous examples and has been given the nomenclature NCIB 11933/GpENT-2ml

Inoculum development stages

i) Ampoule

ii) Slope

iii) Shake flask/florence flask stage

i) Ampoules of the freeze dried strain were prepared from a slope and stored at room temperature.

ii) Slope stage - 0.2 ml sterile LPSG or nutrient broth (NB) medium was added to an ampoule of the bacteria. The contents were asceptically transferred onto nutrient agar (NA) containing 25 ug/ml tetracycline and incubated overnight (16 h) at 37°C.

5 ml sterile LPSG medium were added to the slope and 0.1 ml portions of this suspension were used to inoculate slopes of NA agar medium containing 25 ug/ml tetracycline. The slopes were incubated overnight (16 h) at 37° and then stored at 4°C for a period not greater than 1 month after which this process was repeated.

iii) Shake flask/Florence flask stage - 5 ml sterile LPSG or NB medium was added to a slope and a suspension of the surface growth made. This suspension was then used to inoculate a florence flask.

5 ml of the slope suspension was used to inoculate 400 ml of LPSG or Glycerol/Holme medium in a 2 litre florence flask. The flask was incubated overnight (16-24h) on a rotary shaker at 250 rev/min with a 2" throw with the temperature being controlled to 37°C.

Fermentation Stage

The florence flask grown culture was used to supply 400 ml (1% (v/v)) of inoculum for the fermentation stage. This was carried out in a 70 L vesel containing 40 litres of LPSG or Glycerol/-Holme medium which had been sterilized at 121°C for 45 mins.

The culture was agitated at a speed of 500 rev/min using 3 x 6 bladed, 12 cm diameter turbing impellers. A constant temperature of 37°C was maintained and sterile air was supplied at a rate of 40 L/min. The fermentation was harvested at 20 h when LPSG medium and at 44 h when Glycerol/Holme medium was used.

Samples were taken at intervals throughout the fermentation for dry weight measurement and LT toxoid determined using an ELISA method as described in Example 1A(ix). Filtered broth samples were used to determine the fermentation parameters which included pH, ammonia, glucose and phosphate.

The results of 2 x 40 litre fermentations using LPSG and Glycerol/Holme medium respectively are shown.

Example A 40 litre scale

| Hours | Dry wt. g/L | Glycerol g/L | pH | LT toxoid mg/L |
|---|---|---|---|---|
| 0 | 0.12 | 35.3 | 6.85 | - |
| 3 | 0.66 | 34.5 | 7.05 | 4 |
| 6 | 5.56 | 30.4 | 7.20 | 24 |
| 9 | 8.89 | 21.3 | 6.70 | 91 |
| 12 | 8.57 | 13.0 | 6.70 | 70 |
| 20 | 8.51 | 2.3 | 8.05 | 119 |

Example B 40 litre scale

| Hours | Dry wt. g/L | Glycerol g/L | pH | LT toxoid mg/L |
|---|---|---|---|---|
| 0 | 0.08 | 34.2 | 6.9 | - |
| 3 | 0.03 | 34.3 | 6.9 | - |
| 6 | 0.09 | 34.4 | 6.8 | - |
| 9 | 0.12 | 33.5 | 6.7 | - |
| 12 | 0.19 | 33.2 | 6.7 | 3.5 |
| 20 | 0.61 | 32.1 | 6.8 | 4.5 |
| 23 | 0.84 | 34.5 | 6.8 | 6 |
| 26 | 1.21 | 35.3 | 6.8 | 9 |
| 29 | 2.04 | 33.0 | 6.5 | 16 |
| 44 | 4.27 | 18.9 | 6.34 | 33.5 |

Media Used

| LPSG | g/l |
|---|---|
| Lab Lemco | 20 |
| peptone | 40 |
| NaCl | 2 |
| Glycerol | 30 |
| pH adjust to 7.0 | |

| Nutrient Agar | g/L |
|---|---|
| Lab Lemco | 10 |
| Peptone | 10 |
| NaCl | 5 |
| Agar No. 3 | 15 |

| Nutrient Broth | g/L |
|---|---|
| Lab Lemco | 10 |
| Peptone | 10 |
| NaCl | 5 |

| <u>Glycerol/Holme</u> | g/l |
|---|---|
| $Na_2HPO_4.12H_2O$ | 37.5 |
| $KH_2PO_4$ | 6.0 |
| $Na_2SO_4$ | 0.5 |
| $NH_4Cl$ | 3.0 |
| $MgSO_4.7H_2O$ | 0.2 |
| L-proline | 0.4 |
| L-leucine | 0.4 |
| Thiamine | 0.5 mg |
| *Trace elements | 1 ml |
| Glycerol | 30.0 |

pH adjust to 7.0

* Trace element solution (g/L) $CaCl_2$ $2H_20$, 0.5; $FeCl_2$ $6H_20$, 16.7; $ZnSO_4$ $7H_20$, 0.18; $CoCl_2$ $6H_2O$, 0.18; NaEDTA, 20.1; $CuSO_4$ $5H_2O$, 0.16; $MnSO_4$ $4H_2O$, 0.15.

Example 5

Vaccines containing LT Toxoid

A) Preparation of whole cells containing LT Toxoid

E.coli NCIB 11933/GpENT - 2ml grown as described in Example 4 were harvested by centrifugation and an aliquot of the cell paste resuspended in TEAN buffer. The suspension was centrifuged at 10,000g for 15 min at 4°C and resuspended in TEAN buffer. Formalin was added to give a final concentration of 0.5% vol/vol. The suspension was incubated at 37°C for 16 hrs, centrifuged at 10,000g for 15 min at 4°C and the cells washed with TEAN buffer. The suspension was recentrifuged at 10,000g for 20 min at 4°C and the pellet resuspended in TEAN to give an LT toxoid concentration that was suitable for vaccine formulation.

B) Preparation of isolated LT toxoid

Purified LT toxoid used in the vaccine was prepared as described in Example 1 viii).

C) Vaccine formulation

Vaccines of whole cells containing LT toxoid or purified LT toxoid were prepared as adjuvant emulsions in Drakeol 6VR oil containing 10% Arlacel 80 oil (vol/vol) and aqueous 4.25% Tween 80 (vol/vol) with the ratio of oil to water being 4:1 (vol/vol). The vaccines also contained 0.02% (vol/vol) thiomerosal.

D) Administration

The vaccines (A) and (B) above were administered as 2ml doses, intramuscularly behind the right ear of the pig at 5 ug/Kg body weight. Two doses were given for a four week interval between doses. ELISA assay showed that antibody titres to all the LT toxoid vaccine formulations remained high for at least 8 weeks after the second injection.

FB 146-917C2

## CLAIMS

1. A toxoid comprising an inactive form of a toxin or toxic component of a toxin, which inactive form is substantially homologous with said toxin or toxic component, is produced by expression of a modified gene and is substantially non-toxic while being, alone or in combination with one or more non-toxic components, capable of stimulating the production of antibodies against the said toxin or toxic component.

2. A toxoid as claimed in claim 1 wherein the active toxin or toxic component of a toxin is causative of acute diarrhoea.

3. A toxoid as claimed in claim 1 or claim 2 wherein the active toxic component is the A component of the heat labile toxin (LT) causative of pig scours.

4. A toxoid as claimed in claim 3 wherein the inactive LTA component is combined with the LTB component to form an inactive form of LT toxin.

5. A vaccine preparation containing a toxoid as claimed in claim 1.

6. A vaccine preparation active against pig scours containing an inactivated LTA component as claimed in claim 3 together with the LTB component, the preparation containing additionally K88 antigens, optionally together with whole cells comprising said antigens and/or whole cells containing said inactivated LTA component.

7. A gene coding for a toxoid as claimed any one of claims 1, 2 or 3.

8. A vector comprising a gene as claimed in claim 7, selected from the group consisting of GpENT-2m1, GpENT-2m2 and GpENT-2m3.

9. A host cell containing a vector as claimed in claim 8.

10. A method of inactivation of a toxin or toxic component of a toxin wherein a gene coding for the toxin or toxic component of the toxin is modified in such a way that when the modified gene, if necessary with other genes, is incorporated into a vector capable of replication in a host organism and used to transform said host organism it produces a toxoid as claimed in claim 1.

11. Use of an inactive form of a toxin or toxic component of a toxin as claimed in claim 1 in combating a disease caused by the said toxin or toxic component.

1 A T G A A A A A T A T A A C T T T C A T T T T T T T T A T T
1 Met Lys Asn Ile Thr Phe Ile Phe Phe Ile

31 T T A T T A G C A T C G C C A T T A T A T G C A A A T G G C
11 Leu Leu Ala Ser Pro Leu Tyr Ala Asn Gly

61 G A C A G A T T A T A C C G T G C T G A C T C T A G A C C C
21 Asp Arg Leu Tyr Arg Ala Asp Ser Arg Pro

91 C C A G A T G A A A T A A A A C G T T C C G G A G G T C T T
31 Pro Asp Glu Ile Lys Arg Ser Gly Gly Leu

121 A T G C C C A G A G G G C A T A A T G A G T A C T T C G A T
41 Met Pro Arg Gly His Asn Glu Tyr Phe Asp

151 A G A G G A A C T C A A A T G A A T A T T A A T C T T T A T
51 Arg Gly Thr Gln Met Asn Ile Asn Leu Tyr

181 G A T C A C G C G A G A G G A A C A C A A A C C G G C T T T
61 Asp His Ala Arg Gly Thr Gln Thr Gly Phe

211 G T C A G A T A T G A T G A C G G A T A T G T T T C C A C T
71 Val Arg Tyr Asp Asp Gly Tyr Val Ser Thr

241 T C T C T T A G T T T G A G A A G T G C T C A C T T A G C A
81 Ser Leu Ser Leu Arg Ser Ala His Leu Ala

271 G G A C A G T C T A T A T T A T C A G G A T A T T C C A C T
91 Gly Gln Ser Ile Leu Ser Gly Tyr Ser Thr

301 T A C T A T A T A T A T G T T A T A G C G A C A G C A C C A
101 Tyr Tyr Ile Tyr Val Ile Ala Thr Ala Pro

331 A A T A T G T T T A A T G T T A A T G A T G T A T T A G G C
111 Asn Met Phe Asn Val Asn Asp Val Leu Gly

361 G T A T A C A G C C C T C A C C C A T A T G A A C A G G A G
121 Val Tyr Ser Pro His Pro Tyr Glu Gln Glu

391 G T T T C T G C G T T A G G T G G A A T A C C A T A T T C T
131 Val Ser Ala Leu Gly Gly Ile Pro Tyr Ser

421 C A G A T A T A T G G A T G G T A T C G T G T T A A T T T T
141 Gln Ile Tyr Gly Trp Tyr Arg Val Asn Phe

451 G G T G T G A T T G A T G A A C G A T T A C A T C G T A A C
151 Gly Val Ile Asp Glu Arg Leu His Arg Asn

481 A G G G A A T A T A G A G A C C G G T A T T A C A G A A A T
161 Arg Glu Tyr Arg Asp Arg Tyr Tyr Arg Asn

511 C T G A A T A T A G C T C C G G C A G A G G A T G G T T A C
171 Leu Asn Ile Ala Pro Ala Glu Asp Gly Tyr

541 A G A T T A G C A G G T T T C C C A C C G G A T C A C C A A
181 Arg Leu Ala Gly Phe Pro Pro Asp His Gln

571 G C T T G G A G A G A A G A A C C C T G G A T T C A T C A T
191 Ala Trp Arg Glu Glu Pro Trp Ile His His

601 G C A C C A C A A G G T T G T G G A A A T T C A T C A A G A
201 Ala Pro Gln Gly Cys Gly Asn Ser Ser Arg

631 A C A A T C A C A G G T G A T A C T T G T A A T G A G G A G
211 Thr Ile Thr Gly Asp Thr Cys Asn Glu Glu

661 A C C C A G A A T C T G A G C A C A A T A T A T C T C A G G
221 Thr Gln Asn Leu Ser Thr Ile Tyr Leu Arg

691 G A A T A T C A A T C A A A A G T T A A G A G G C A G A T A
231 Glu Tyr Gln Ser Lys Val Lys Arg Gln Ile

721 T T T T C A G A C T A T C A G T C A G A G G T T G A C A T A
241 Phe Ser Asp Tyr Gln Ser Glu Val Asp Ile

751 T A T A A C A G A A T T C G G G A T G A A T T A T G A
251 Tyr Asn Arg Ile Arg Asp Glu Leu End

FIG. 1.

```
  1 A T G A A A A A T A T A A C T T T C A T T T T T T T T A T T
  1 Met   Lys   Asn   Ile   Thr   Phe   Ile   Phe   Phe   Ile

 31 T T A T T A G C A T C G C C A T T A T A T G C A A A T G G C
 11 Leu   Leu   Ala   Ser   Pro   Leu   Tyr   Ala   Asn   Gly

 61 G A C A G A T T A T A C C G T G C T G A C T C T A G A C C C
 21 Asp   Arg   Leu   Tyr   Arg   Ala   Asp   Ser   Arg   Pro

 91 C C A G A T G A A A T A A A A C G T T C C G G A G G T C T T
 31 Pro   Asp   Glu   Ile   Lys   Arg   Ser   Gly   Gly   Leu

121 A T G C C C A G A G G G C A T A A T G A G T A C T T C G A T
 41 Met   Pro   Arg   Gly   His   Asn   Glu   Tyr   Phe   Asp

151 A G A G G A A C T C A A A T G A A T A T T A A T C T T T A T
 51 Arg   Gly   Thr   Gln   Met   Asn   Ile   Asn   Leu   Tyr

181 G A T C A C G C G A G A G G A A C A C A A A C C G G C T T T
 61 Asp   His   Ala   Arg   Gly   Thr   Gln   Thr   Gly   Phe

211 G T C A G A T A T G A T G A C G G A T A T G T T |T C C |A C T
 71 Val   Arg   Tyr   Asp   Asp   Gly   Tyr   Val   |Ser   |Thr
                                                    |T T C |
                                                    |Phe   |
                                                    |SDM  1|

241 T C T C T T A G T T T G A G A A G T G C T C A C T T A G C A
 81 Ser   Leu   Ser   Leu   Arg   Ser   Ala   His   Leu   Ala

271 G G A C A G T C T A T A T T A T C A G G A T A T T C C A C T
 91 Gly   Gln   Ser   Ile   Leu   Ser   Gly   Tyr   Ser   Thr

301 T A C T A T A T A T A T G T T A T A G C G A C A G C A C C A
101 Tyr   Tyr   Ile   Tyr   Val   Ile   Ala   Thr   Ala   Pro

331 A A T A T G T T T A A T G T T A A T G A T G T A T T A G G C
111 Asn   Met   Phe   Asn   Val   Asn   Asp   Val   Leu   Gly

361 G T A T A C A G C C C T C A C C C A T A T G A A C A G G A G
121 Val   Tyr   Ser   Pro   His   Pro   Tyr   Glu   Gln   Glu

391 G T T T C T G C G T T A G G T G G A A T A C C A T A T T C T
131 Val   Ser   Ala   Leu   Gly   Gly   Ile   Pro   Tyr   Ser

421 C A G A T A T A T G G A T G G T A T C G T G T T A A T T T T
141 Gln   Ile   Tyr   Gly   Trp   Tyr   Arg   Val   Asn   Phe

451 G G T G T G A T T G A T G A A C G A T T A C A T C G T A A C
151 Gly   Val   Ile   Asp   Glu   Arg   Leu   His   Arg   Asn

481 A G G G A A T A T A G A G A C C G G T A T T A C A G A A A T
161 Arg   Glu   Tyr   Arg   Asp   Arg   Tyr   Tyr   Arg   Asn

511 C T G A A T A T A G C T C C G G C A G A G G A T G G T T A C
171 Leu   Asn   Ile   Ala   Pro   Ala   Glu   Asp   Gly   Tyr

541 A G A T T A G C A G G T T T C C C A C C G G A T C A C C A A
181 Arg   Leu   Ala   Gly   Phe   Pro   Pro   Asp   His   Gln

571 G C T T G G A G A G A A G A A C C C T G G A T T C A T C A T
191 Ala   Trp   Arg   Glu   Glu   Pro   Trp   Ile   His   His

601 G C A C C A C A A G G T T G T G G A A A T T C A T C A A G A
201 Ala   Pro   Gln   Gly   Cys   Gly   Asn   Ser   Ser   Arg

631 A C A A T C A C A G G T G A T A C T T G T A A T G A G G A G
211 Thr   Ile   Thr   Gly   Asp   Thr   Cys   Asn   Glu   Glu

661 A C C C A G A A T C T G A G C A C A A T A T A T C T C A G G
221 Thr   Gln   Asn   Leu   Ser   Thr   Ile   Tyr   Leu   Arg

691 G A A T A T C A A T C A A A A G T T A A G A G G C A G A T A
231 Glu   Tyr   Gln   Ser   Lys   Val   Lys   Arg   Gln   Ile

721 T T T T C A G A C T A T C A G T C A G A G G T T G A C A T A
241 Phe   Ser   Asp   Tyr   Gln   Ser   Glu   Val   Asp   Ile

751 T A T A A C A G A A T T C G G G A T G A A T T A T G A
251 Tyr   Asn   Arg   Ile   Arg   Asp   Glu   Leu   End
```

FIG. 2.

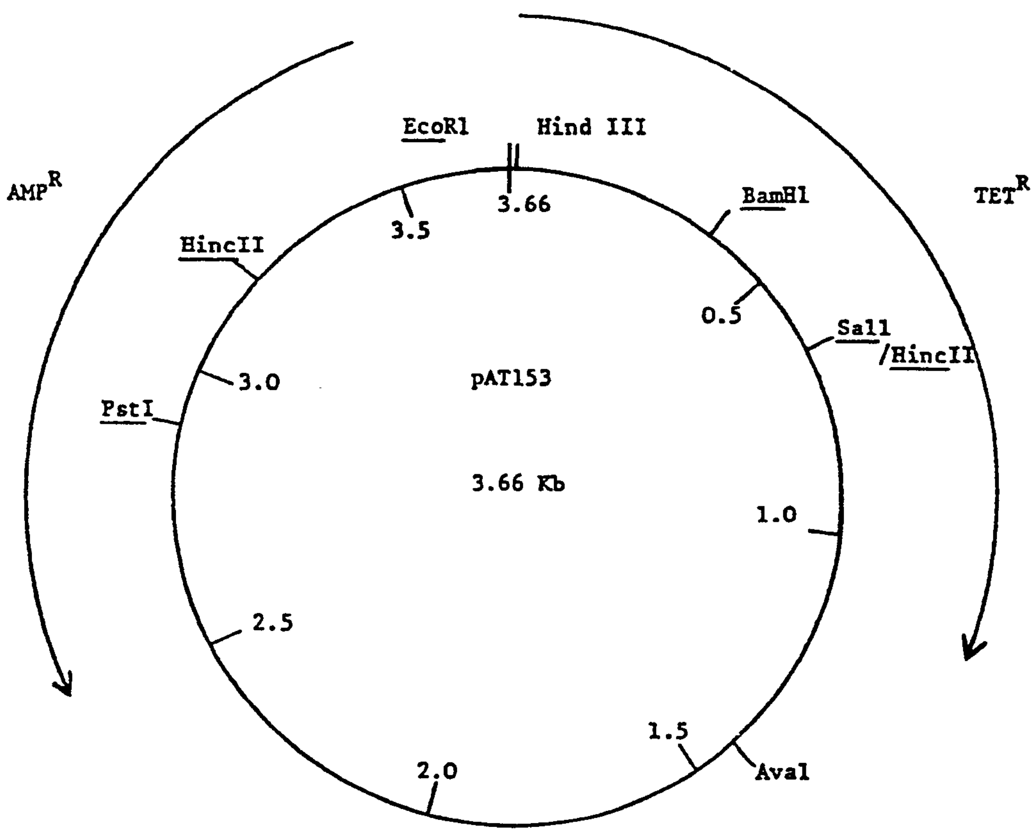

EcoR1
Hind III
AMP^R
TET^R
3.66
3.5
BamH1
HincII
0.5
Sal1
/HincII
3.0
PstI
pAT153
3.66 Kb
1.0
2.5
1.5
AvaI
2.0


FIG. 3.

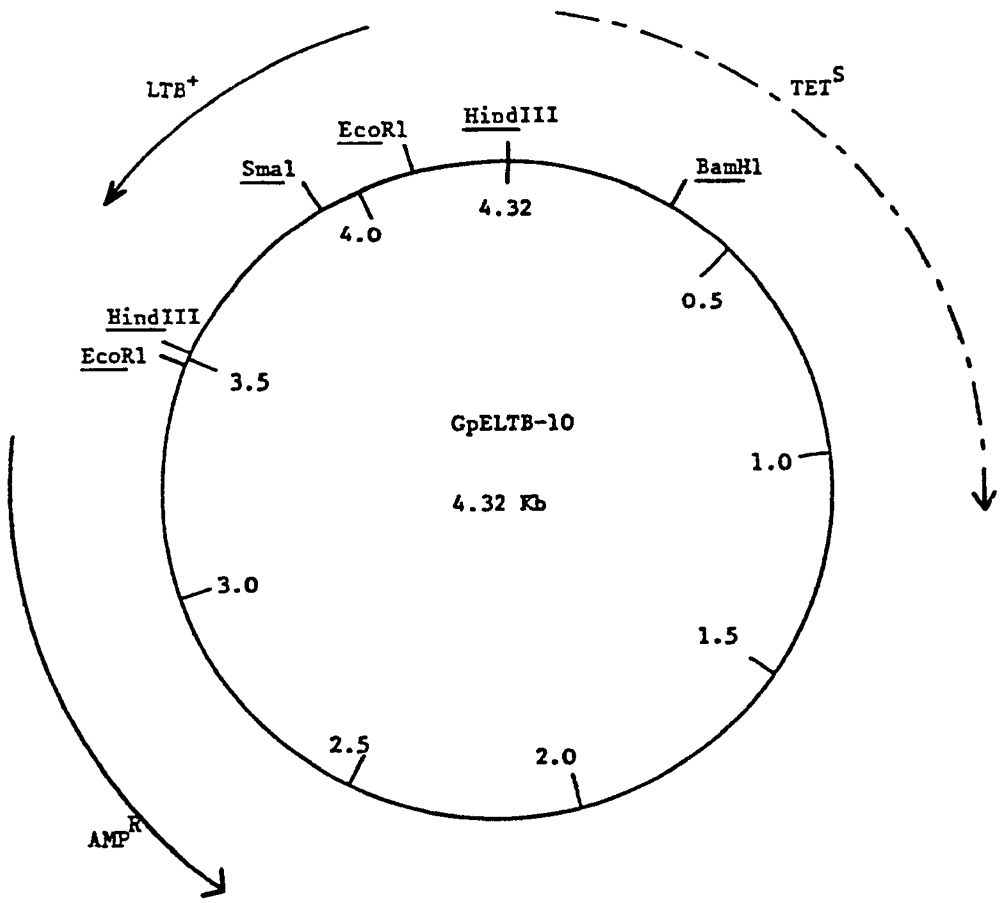
LTB+
TETS
EcoR1
HindIII
Sma1
BamH1
4.32
4.0
0.5
HindIII
EcoR1
3.5
GpELTB-10
4.32 Kb
1.0
3.0
1.5
2.5
2.0
AMPR

FIG. 4.

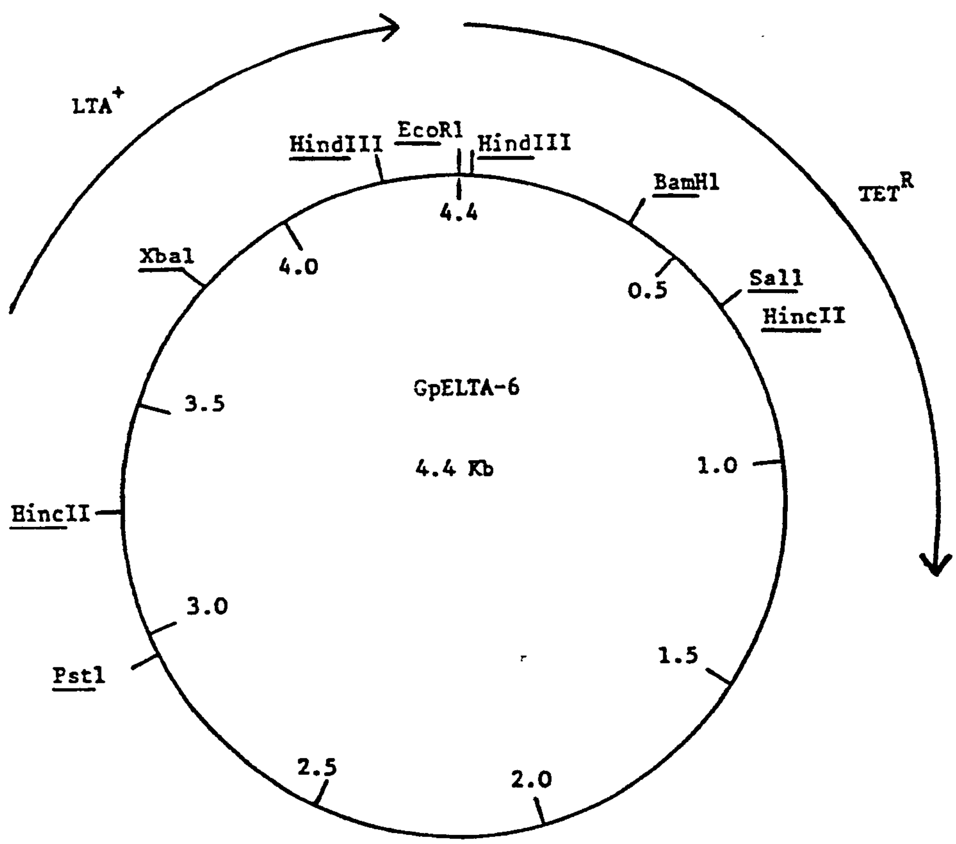
LTA+
TETR
HindIII
EcoR1
HindIII
4.4
BamH1
0.5
Sal1
HincII
Xba1
4.0
GpELTA-6
4.4 Kb
3.5
1.0
HincII
3.0
Pst1
1.5
2.5
2.0

FIG. 5.

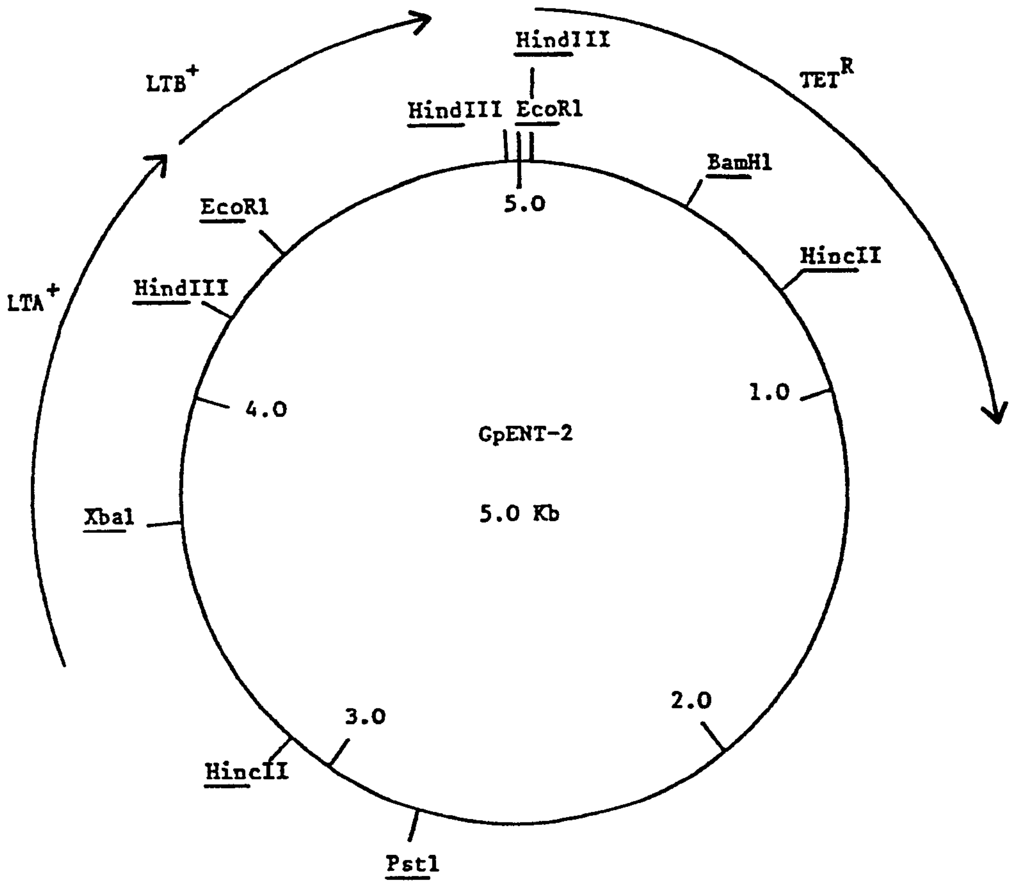
LTB+
HindIII
TETR
HindIII EcoR1
BamH1
EcoR1
5.0
HincII
LTA+
HindIII
1.0
4.0
GpENT-2
5.0 Kb
Xba1
2.0
3.0
HincII
Pst1

FIG. 6.

```
                          XbaI
a) 5' -G C T G A C T C T A G A C C C C C A
       C G A C T G A G A T C T G G G G G T - 5'
b)     —Ala—Asp— Ser—Arg—Pro—Pro—
```

FIG. 7.

```
c) 5'    C T A G T C C G C G G A
               A G G C G C C T G A T C    5'
```

```
                                  SstII
d) 5' - G C T G A C T C T A G T C C G C G G A C T A G A C C C C C A
        C G A C T G A G A T C A G G C G C C T G A T C T G G G G G T  - 5'
e)     - Ala — Asp — Ser — Ser — Pro — Arg — Thr — Arg — Pro — Pro —
```

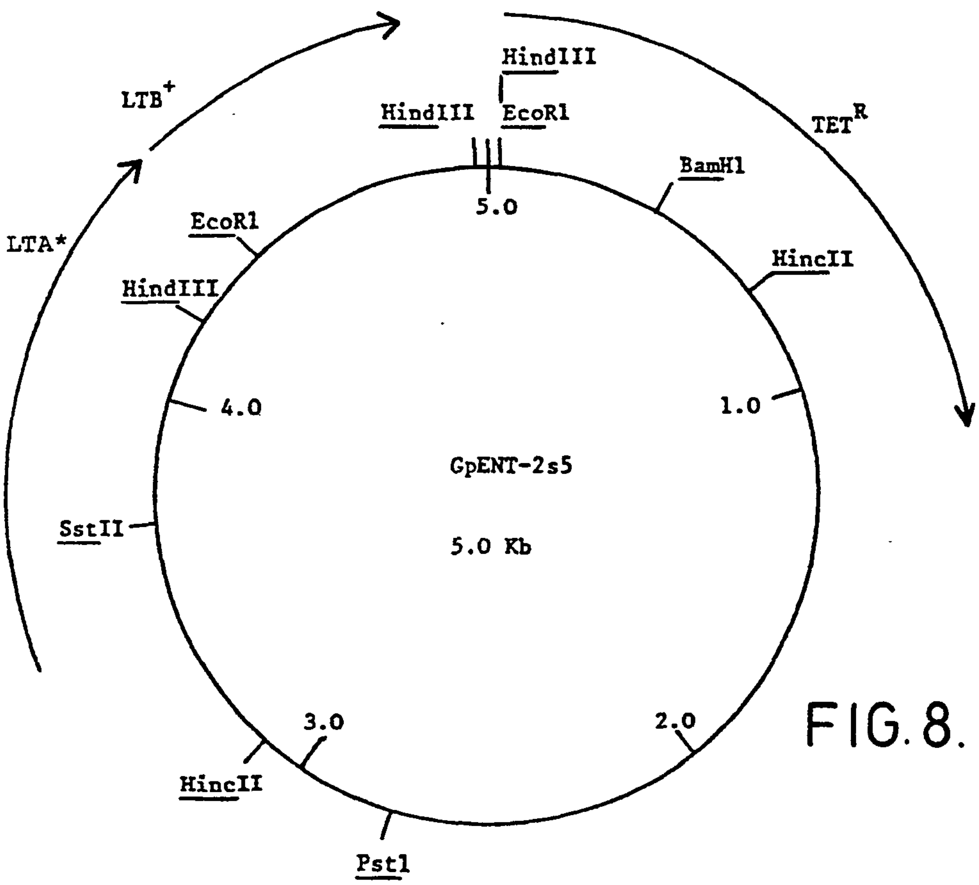


FIG. 8.